# EUROPÄISCHE PATENTSCHRIFT

(11) **EP 1 816 154 B1**
(45) Veröffentlichungstag und Bekanntmachung des Hinweises auf die Patenterteilung: **13.04.2011**
(21) Anmeldenummer: 07001258.8
(22) Anmeldetag: 20.01.2007
(51) Int. Cl.: C08G 77/14, C12P 7/64

(54) **Verfahren zur Herstellung organomodifizierter Siloxane**
Method for producing organomodified siloxanes
Procédé de fabrication de siloxane organomodifié

(30) Priorität: 04.02.2006 DE 102006005100
(43) Veröffentlichungstag der Anmeldung: 08.08.2007
(73) Patentinhaber: Evonik Goldschmidt GmbH, 45127 Essen (DE)
(72) Erfinder: Ferenz, Michael, Dr., 45147 Essen (DE); Grüning, Burghard, Dr., 45134 Essen (DE); Hartung, Christian, Dr., 45133 Essen (DE); Thum, Oliver, Dr., 40880 Ratingen (DE)

(56) Entgegenhaltungen:
- EP-A- 0 407 959
- EP-A1- 1 250 842
- EP-A2- 0 965 645
- WO-A-2004/099290
- JP-A- 8 157 601
- US-A- 6 121 347
- US-A1- 2003 096 919

## Beschreibung

Die Erfindung betrifft ein neues Verfahren zur Herstellung von mit organischen Gruppen modifizierten Siloxanen.

Organomodifizierte Siloxane werden in den verschiedensten Applikationen eingesetzt. Ihre Eigenschaften lassen sich durch die Art der Modifikation, sowie durch die Modifikationsdichte gezielt einstellen.

Eine industriell genutzte Methode zur Produktion von organomodifizierten Siloxanen ist die Hydrosilylierung. Dazu werden Si-H-funktionelle Siloxane in Gegenwart von Übergangsmetallkatalysatoren mit endständig ungesättigten organischen Reaktionspartnern umgesetzt.

So können zum Beispiel mit Allylpolyethern organophile oder nichtionische hydrophile Gruppen an ein Siloxangerüst gebunden werden. Derartige Verbindungen finden ihren Einsatz zum Beispiel als Polyurethan-Schaum-Stabilisatoren, als Entschäumer in Treibstoffen oder als Additive in Farben und Lacken.

Durch Umsetzung mit α-Olefinen wird dagegen das Siloxan mit hydrophoben Gruppen verknüpft. Die so erhaltenen Siliconwachse dienen zum Beispiel als Additiv in Personal-Care-Applikationen.

Es zeigt sich in vielen Anwendungsgebieten, dass die Wirkung des Siloxans entscheidend von der Verträglichkeit mit der entsprechenden Formulierung abhängt. Es ist daher wünschenswert, auf eine breite Rohstoffbasis an organischen Resten zurückgreifen zu können, die sich außerdem mit Präzision und sehr guten Ausbeuten reproduzierbar durch Hydrosilylierung an das Silicongrundgerüst addieren lässt.

Eine sehr breite und in vielen Fällen zudem recht preiswerte Rohstoffbasis stellt die Gruppe der Carbonsäuren dar. Die verfügbaren Produkte sind zahlreich und unterscheiden sich gravierend in ihren Eigenschaften. Beispielhaft sei hier die Klasse der Fettsäuren genannt.

Carbonsäuren lassen sich auch in Form ihrer Ester an das Siloxangerüst knüpfen. Solchermaßen mit organischen Estergruppen modifizierte Siloxane sind über verschiedene Syntheserouten zugänglich und finden Anwendung beispielsweise als Additive für Toner.

Zum einen können Alkoholgruppen tragende Siloxane verestert oder Carbonsäureester mit Alkoholgruppen tragenden Siloxanen umgeestert werden.

Diese beiden Verfahren setzen jedoch voraus, dass zunächst ein Alkoholgruppen tragendes Siloxan synthetisiert wird, was in vielen Fällen nicht unproblematisch ist. Zugänglich sind letztere durch Hydrosilylierung von endständig ungesättigten Alkoholen, wie zum Beispiel Allylalkohol oder Glycerinmonoallylether. Während der Hydrosilylierung kommt es jedoch in der Regel zu Nebenreaktionen, bei der die SiH-Einheiten mit der OH-Funktion unter Bildung von SiOC-Gruppen reagieren. Insbesondere bei hochmodifizierten Siloxanen kommt es daher schnell zu einer unkontrollierten und unerwünschten Vernetzung der Reaktionsprodukte.

Weiterhin ist die Umsetzung der so gewonnenen Alkoholgruppen tragenden Siloxane zu den entsprechenden Estern ebenfalls häufig problematisch, da viele der üblicherweise angewandten Reaktionsbedingungen zur Ver- bzw. Umesterung, beispielsweise der Einsatz von starken Säuren bei Temperaturen von über 100 °C, zu Umlagerungsreaktionen im Siloxangerüst führen.

Ein weiterer Weg, Carbonsäureester funktionalisierte Siloxane zu erhalten, ist die Hydrosilylierung von endständig ungesättigten Estern. Die Patentanmeldung JP-A-8157601 beschreibt Alkenylester und Allyloxyethanolester, aber keine Allylpolyglycolester. JP-A-8157601 und US-A-2003/0096919 beschreiben zwar den Einsatz terminal ungesättigter Ester, machen jedoch keine Angaben über die Herstellung und ggf. Aufreinigung dieser Verbindungen. Da die Hydrosilylierung aber eine sehr empfindliche Reaktion ist, sind teilweise umfangreiche Reinigungsschritte nötig, um Ester zu erhalten, die problemlos weiter mit Siloxanen umgesetzt werden können. Aus wirtschaftlichen und toxikologischen Gründen ist dabei eine niedrige Katalysatorkonzentration, üblicherweise unter 15 ppm, wünschenswert, wodurch ein problemloser Reaktionsverlauf jedoch noch schwerer zu erreichen ist.

Die bekannten konventionellen Methoden zur industriellen Herstellung von Estern, wie sie beispielsweise in der Herstellung kosmetischer Ester häufig verwendet werden, beinhalten den Einsatz von Säuren oder Metallsalzen als Katalysatoren.

Allerdings liefern endständig ungesättigte Ester die nach derartigen Standardmethoden, beispielsweise durch Säurekatalyse mit para-Toluensulfonsäure oder durch Metallsalzkatalyse, mit z. B. Zinn(II)oxalat, hergestellt wurden, nur unbefriedigende Ergebnisse in der Hydrosilylierung, wenn die verwendeten Katalysatoren lediglich durch übliche einfache Verfahren, wie Neutralisation und Filtration abgetrennt wurden. Die später folgenden Beispiele 1, 2, 11 und 12 belegen dies durch experimentelle Daten.

Eine Aufgabe der vorliegenden Erfindung bestand daher darin, ein Verfahren zu entwickeln, welches zunächst die einfache Herstellung von terminal ungesättigten organischen Estern erlaubt, die anschließend problemlos und ohne nennenswerte Aufarbeitungsschritte durch Hydrosilylierung mit Siloxanen verknüpft werden können.

Weitere nicht explizit genannte Aufgaben ergeben sich aus dem Kontext der nachfolgenden Beschreibung, der Beispiele sowie der Ansprüche.

Überraschenderweise wurde gefunden, dass organische Ester, die eine terminale Doppelbindung enthalten, einfach und in sehr guten Ausbeuten durch Hydrosilylierung an Siloxane angelagert werden können, wenn die Knüpfung der Esterbindung durch enzymatische Katalyse erfolgt.

Gegenstand der Erfindung ist daher ein Verfahren zur Herstellung von mit organischen Estern modifizierten Siloxanen, welches dadurch gekennzeichnet ist, dass die eingesetzten terminal ungesättigten Ester der allgemeinen Formel I 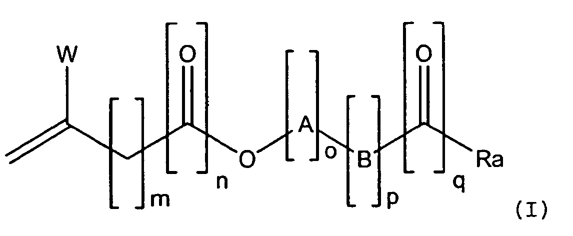 wobei
- W: Wasserstoff oder Methyl, bevorzugt Wasserstoff,
- m: 0 bis 28, bevorzugt 1 bis 17,
- n: 0 oder 1,
- o: 0 bis 100,
- A: ein Oxyalkenylrest der allg. Formel Ia 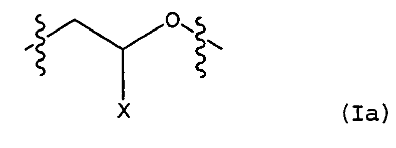
ist, wobei
- X: unabhängig voneinander gleiche oder verschiedene Reste aus der Gruppe -H, -CH₃, -CH₂CH₃ oder -Phenyl, bevorzugt Wasserstoff, ist,
- p: 0 bis 20,
- B: für p = 1, ein vom Glycerin abgeleiteter, gegebenenfalls mit R_{b} veresterter, Oxyalkenylrest der allg. Formel Ib 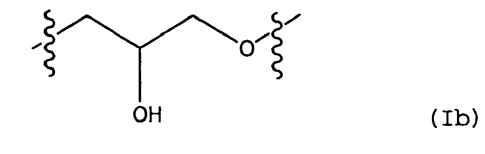
ist, wobei
- R_{b}: der Acylrest linearer, verzweigter, gesättigter oder ungesättigter, gegebenenfalls zusätzliche Hydroxygruppen tragender Carbonäuren mit 2 bis 30 C-Atomen ist, oder
- B: für p = 1, der Rest der allg. Formel Ic 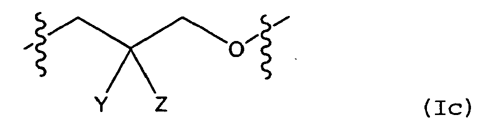
ist, wobei
- Y und Z: unabhängig voneinander gleiche oder verschiedene Reste aus der Gruppe -H, -CH₃, -CH₂CH₃, -CH₂OH oder -CH₂OR_{b} sind,
wobei
R_{b} wie oben definiert ist, oder
- B: für p ≥ 2, ein vom Polyglycerin abgleiteter, gegebenenfalls mit R_{b} veresterter, Rest ist,
wobei
R_{b} wie oben definiert ist,
- Rₐ: Wasserstoff, Rₐ₁ oder Rₐ₂, ist,
wobei
Rₐ₁ ein linearer oder verzweigter, gesättigter oder ungesättigter, gegebenenfalls Hydroxygruppen tragender, gegebenenfalls mit Carbonsäuren veresterter Hydroxygruppen tragender, gegebenenfalls Amino-, Alkylamino- oder Dialkylaminogruppen tragender Alkylrest mit 1 bis 200 C-Atomen,
Rₐ₂ der Rest der allgemeinen Formel Id 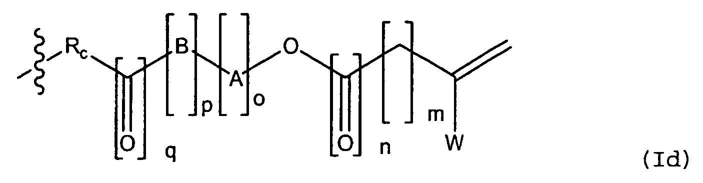 ist, und
R_{c} ein gesättigter oder ungesättigter difunktioneller Kohlenwasserstoffrest mit 2 bis 20 C-Atomen ist, wobei
n + q 1 und wenn
Rₐ Wasserstoff ist, dann ist
q 0 und
o + p ≥ 1,
biokatalytisch hergestellt werden.

Erfindungsgemäß verwendet werden bevorzugt Verbindungen der allgemeinen Formel I, bei denen
n,o,p = 0,
- W: Wasserstoff,
- m: 1 bis 28, bevorzugt 1 bis 4,
- q: 1,
Rₐ, Rₐ₁= Rₐ₁₁ oder Rₐ = Rₐ₂ ist.

Rₐ₁₁ stellt dabei den von handelsüblichen Säuren, wie beispielsweise Essigsäure, Propansäure, Butansäure, Pentansäure, Chloressigsäure, Trifluoressigsäure, Ethylhexansäure, Isononansäure, Isotridecansäure oder Isostearinsäure abgeleiteten Alkylrest dar. Weiterhin stellt Rₐ₁₁ den von einbasischen Fettsäuren auf Basis natürlicher pflanzlicher oder tierischer Öle mit 6 bis 30 Kohlenstoffatomen, insbesondere mit 8 bis 22 Kohlenstoffatomen, wie Capronsäure, Caprylsäure, Caprinsäure, Laurinsäure, Myristinsäure, Palmitinsäure, Palmitoleinsäure, Isostearinsäure, Stearinsäure, 12-Hydroxystearinsäure, Dihydroxystearinsäure, Ölsäure, Linolsäure, Petrolesinsäure, Elaidinsäure, Arachinsäure, Behensäure, Erucasäure, Gadoleinsäure, Linolensäure, Eicosapentaensäure, Docosahexaensäure, Arachidonsäure, welche allein oder in Mischung eingesetzt werden können, abgeleiteten Alkylrest dar. Als Rest Rₐ₁₁ kann ebenfalls der von Polykondensationsprodukten von hydroxyfunktionalisierten Säuren, beispielsweise Poly-12-hydroxystearinsäure oder Polyricinolsäure, abgeleitete Alkylrest eingesetzt werden.

Erfindungsgemäß ebenfalls bevorzugt eingesetzt werden Verbindungen der allgemeinen Formel I, bei denen
n,p 0,
- W: Wasserstoff,
- m: 1 bis 28, bevorzugt 1 bis 4, insbesondere 1,
- o: 1 bis 100, insbesondere 1,
- q: 1, ist,
- Rₐ: Rₐ₁ = Rₐ₁₁ oder Rₐ = Rₐ₂ ist, wobei Rₐ₁₁ und Rₐ₂ wie oben definiert sind.

Erfindungsgemäß ebenfalls bevorzugt eingesetzt werden Ester der allgemeinen Formel I, wobei
n,o 0,
- W: Wasserstoff,
- m: 1 bis 28, bevorzugt 1 bis 4, insbesondere 1,
- B: -CH₂-CH₂OH-CH₂-O- oder -CH₂-CH₂OR_{b}-CH₂O-,
- P: 1 bis 20, bevorzugt 1,
- q: 1, ist,
- Rₐ: Rₐ₁ = Rₐ₁₁ oder Rₐ = Rₐ₂, ist
wobei
Rₐ₁₁, Rₐ₂ und R_{b} wie oben definiert sind.

Hierbei können die Polyglycerinderivate (p ≥ 2) zumindest teilweise andere, als aus Formel Ib ersichtlchen 1,3-Verknüpfungen der Glycerineinheiten aufweisen, wie sie in den üblichen und literaturbekannten Verfahren zur Gewinnung von Polyglycerinen auftreten, beispielsweise 1,2-Verknüpfungen.

Erfindungsgemäß ebenfalls bevorzugt eingesetzt werden Ester der allgemeinen Formel I, wobei
n,o 0,
- W: Wasserstoff,
- m: 1 bis 28, bevorzugt 1 bis 4, insbesondere 1,
- B: -CH₂-CH₂YZ-CH₂-O-,
- P: q = 1,
- Rₐ: Rₐ₁ = Rₐ₁₁ oder Rₐ = Rₐ₂, wobei Y, Z, Rₐ₁₁ und Rₐ₂ wie oben definiert sind.

Erfindungsgemäß ebenfalls bevorzugt eingesetzt werden Ester der allgemeinen Formel I, wobei
n 1,
- W: Wasserstoff oder CH₃, bevorzugt Wasserstoff,
- m: 0 bis 27, bevorzugt 1 bis 10,
- o,p: 0,
- Rₐ: Rₐ₁ = Rₐ₁₂ oder Rₐ = Rₐ₂, wobei Rₐ₂ wie oben definiert sind und
- Rₐ₁₂: der Kohlenwasserstoffrest eines substituierten oder unsubstituierten, gegebenenfalls verzweigten, gegebenenfalls eine oder mehrere Mehrfachbindung(en) enthaltenden, gegebenenfalls Hydroxygruppen, gegebenenfalls mit Carbonsäuren veresterte Hydroxygruppen, gegebenenfalls Amino-, Alkylamino- oder Dialkylaminogruppen tragenden Alkohols mit 2 bis 30 C-Atomen, bevorzugt mit 6 bis 22 C-Atomen, ist.

Beispiele für Rₐ₁₂ sind die Kohlenwasserstoffreste von Propanol, Butanol, Pentanol, Hexanol, Octanol sowie deren Isomeren wie i-Propanol, i-Butanol, 2-Ethylhexanol, Isononylalkohol, Isotridecylalkohol, mehrwertige Alkohole, wie 1,6-Hexandiol, 1,2-Pentandiol, Dihydroxyaceton, 1,2-Propylenglycol, 1,3-Propylenglykol, Neopentylglycol, Trimethylolpropan, Pentaerithrol, Sorbitol, Glycerin, Diglycerin, Triglycerin, Polyglycerin, Ethylenglykol, Diethylenglycol, Triethylenglycol, Polyethylenglycol, sowie aminofunktionalisierte Alkohole, wie N,N-Dimethylethanolamin. Weitere Beispiele sind die Kohlenwasserstoffreste von Alkoholen, die nach bekannten Verfahren aus einbasischen Fettsäuren auf Basis natürlicher pflanzlicher oder tierischer Öle mit 6 bis 30 Kohlenstoffatomen, insbesondere mit 8 bis 22 Kohlenstoffatomen, hergestellt werden, wie Capronsäure, Caprylsäure, Caprinsäure, Laurinsäure, Myristinsäure, Palmitinsäure, Palmitoleinsäure, Isostearinsäure, Stearinsäure, 12-Hydroxystearinsäure, Dihydroxystearinsäure, Ölsäure, Linolsäure, Petroselinsäure, Elaidinsäure, Arachinsäure, Behensäure, Erucasäure, Gadoleinsäure, welche allein oder in Mischung eingesetzt werden.

Erfindungsgemäß werden die Ester der allgemeinen Formel I durch Kondensation der entsprechenden Alkohole und Säuren unter Verwendung mindestens eines Enzyms als Katalysator hergestellt. Erfindungsgemäß können statt der Säuren auch die entsprechenden Ester der Carbonsäuren mit leichtflüchtigen Alkoholen zu einer Umesterung eingesetzt werden, beispielsweise eignen sich Methyl-, Ethyl- oder Vinylester.

Bei den erfindungsgemäß verwendbaren Enzymen handelt es sich um solche aus der Gruppe der hydrolytischen Enzyme, z. B. Lipasen, Esterasen oder Proteasen, wie beispielsweise Cholesterol Esterase, Esterase aus Schweineleber oder Lipasen aus *Candida rugosa, Pseudomonas sp., Thermomyces langosiosus,* Schweinepankreas, *Mucor miehei, Alcaligines sp.,* vorzugsweise Lipasen, besonders bevorzugt Lipase B aus *Candida antarctica.*

Erfindungsgemäß können ganze Zellen, ruhende Zellen, immobilisierte Zellen, gereinigte Enzyme oder Zellextrakte, die die entsprechenden Enzyme enthalten oder Mischungen davon eingesetzt werden. Die Enzyme können erfindungsgemäß in Ganzzellsystemen, in freier Form oder auf geeignete Träger immobilisiert verwendet werden.

Bei dem erfindungsgemäßen Verfahren werden die Reaktanden in einem geeigneten Reaktor (z. B. Rundkolben mit Rührer oder in einem Festbettreaktor) gemischt und auf die optimale Arbeitstemperatur des verwendeten Biokatalysators erhitzt. Je nach verwendetem Biokatalysator liegt diese Temperatur bei 20 °C bis 100 °C, bevorzugt bei 35 °C bis 80 °C. Bei Verwendung eines Festbettreaktors wird das Festbett mit dem ausgewählten Enzym befüllt und nach Erreichen der Reaktionstemperatur die Reaktionsmischung durch das Festbett gepumpt. Bei Verzicht auf einen Festbettreaktor wird das Enzym der Reaktionsmischung direkt zugesetzt und nach Beendigung der Reaktion durch geeignete Vorrichtungen abfiltriert. Zum Erreichen möglichst vollständiger Umsätze wird das bei der Reaktion freiwerdende Wasser, bzw. der freiwerdende niedrigsiedende Alkohol durch Anlegen eines Vakuums oder andere geeignete Techniken, beispielsweise Durchleiten inerter Gase (z. B. Stickstoff) oder der Anwendung von Absorptionsmitteln (z. B. Molekularsieb), entfernt.

Erfindungsgemäß werden anschließend die solchermaßen erhaltenen Ester, eventuell auch im Gemisch untereinander und/oder mit anderen, terminal ungesättigten organischen Verbindungen, beispielsweise Allyloxyethanol, Glycerinmonoallylether, Allyltrimethylolpropan, α-Olefinen oder endständig ungesättigten Polyethern, durch Hydrosilylierung mit einem SiH-Polysiloxan der allgemeinen Formel II 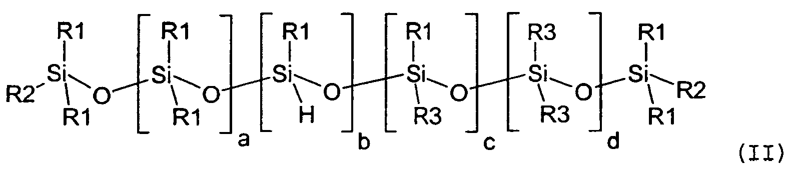 wobei
- N: a + b + c + d + 2 = 3 bis 850, vorzugsweise 6 bis 160,
- a: 1 bis 800, vorzugsweise 2 bis 150,
- b: 0 bis 400, vorzugsweise 2 bis 75,
- c: 0 bis 10, vorzugsweise 0,
- d: 0 bis 10, vorzugsweise 0, ist,
nach bekannten Methoden umgesetzt,
worin die Reste
- R1: unabhängig voneinander gleich oder verschieden und aus folgender Gruppe sind: gesättigte oder ungesättigte, gegebenenfalls verzweigte Alkylgruppen mit 1 bis 30 C-Atomen, Alkarylreste mit 7 bis 30 C-Atomen, Arylreste mit 6 bis 30 C-Atomen, vorzugsweise Alkylgruppen mit 1 bis 4 C-Atomen oder Phenyl, insbesondere Methyl;
- R2: unabhängig voneinander Wasserstoff oder R1;
- R3: unabhängig voneinander gleiche oder verschiedene Reste der allgemeinen Formel IIa 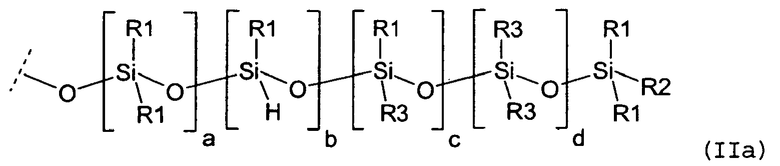
sind.

In einer besonderen Ausführungsform wird das Si-H-Polysiloxan allein oder teilweise mit Estern umgesetzt, die mehr als eine endständige H₂C=CH-Gruppe enthalten. Diese Ester entsprechen der allgemeinen Formel I, in der Rₐ₂ durch die Formel Id beschrieben wird. Auf diese Weise werden höhermolekulare, in der Regel vernetzte organomodifizierte Siloxane erhalten.

Es ist dem Fachmann geläufig, dass die Verbindungen in Form eines Gemisches mit einer im Wesentlichen durch statistische Gesetze geregelten Verteilung vorliegen. Die Werte für die Indices a, b, c und d stellen deshalb Mittelwerte dar.

Erfindungsgemäß wird die Hydrosilylierung nach etablierten Methoden in Gegenwart eines Katalysators durchgeführt. Dabei können beispielsweise Katalysatoren verwendet werden, die üblicherweise für Hydrosilylierungen eingesetzt werden, wie Platin-, Rhodium-, Osmium-, Ruthenium-, Palladium-, Iridium-Komplexe oder ähnliche Verbindungen bzw. die entsprechenden reinen Elemente oder deren auf Silica, Aluminiumoxid oder Aktivkohle oder ähnlichen Trägermaterialien immobilisierte Derivate. Bevorzugt wird die Hydrosilylierung in Gegenwart von Pt-Katalysatoren wie Cis-Platin oder Karstedt-Katalysator [Tris(divinyltetramethyldisiloxan)bis-platin] durchgeführt.

Die bevorzugt eingesetzte Menge an Katalysator beträgt 10⁻⁷ bis 10⁻¹ mol pro mol Olefin, bevorzugt 1 bis 20 ppm. Die Hydrosilylierung wird bei Temperaturen zwischen 0 und 200 °C, bevorzugt zwischen 50 und 140 °C, durchgeführt. Lösungsmittel sind für die Durchführung der Reaktion generell nicht nötig. Die Reaktion kann jedoch in geeigneten Lösungsmitteln wie aliphatischen oder aromatischen Kohlenwasserstoffen, cyclischen Oligosiloxanen, Alkoholen oder Estern durchgeführt werden.

Gegenstand der Erfindung sind weiterhin Verbindungen der Formel I, wobei n = p = 0, W = X = Wasserstoff, m = o = q = 1 und Rₐ = Rₐ₁₃ ist. Dabei ist Rₐ₁₃ der Alkylrest einbasischer Fettsäuren auf Basis natürlicher pflanzlicher oder tierischer Öle mit 8 bis 16 Kohlenstoffatomen, welche allein oder in Mischung vorhanden sein können. Erfindungsgemäß kann Rₐ₁₃ auch der Alkylrest von Isononansäure (3,5,5-Trimethylhexansäure), 2-Ethylhexansäure, Ricinolsäure, 12-Hydroxystearinsäure, Polyricinolsäure oder Poly-12-hydroxystearinsäure sein.

Weiterhin Gegenstand der Erfindung sind Verbindungen der Formel I, wobei n = p = 0, W = Wasserstoff, X = Wasserstoff oder Methyl, bevorzugt Wasserstoff, m = q = 1, o = 2 bis 100, bevorzugt 3 bis 100, und Rₐ = Rₐ₁₄ ist. Dabei ist Rₐ₁₄ der Alkylrest handelsüblicher Säuren mit 4 bis 30 Kohlenstoffatomen, wie beispielsweise Butansäure oder Pentansäure, weiterhin die Alkylreste einbasischer Fettsäuren auf Basis natürlicher pflanzlicher oder tierischer Öle mit 6 bis 30 Kohlenstoffatomen, insbesondere mit 8 bis 22 Kohlenstoffatomen, wie Capronsäure, Caprylsäure; Caprinsäure, Laurinsäure, Myristinsäure, Palmitinsäure, Palmitoleinsäure, Isostearinsäure, Stearinsäure, 12-Hydroxystearinsäure, Dihydroxystearinsäure, Ölsäure, Linolsäure, Petrolesinsäure, Elaidinsäure, Arachinsäure, Behensäure, Erucasäure, Gadoleinsäure, Linolensäure, Eicosapentaensäure, Docosahexaensäure, Arachidonsäure, welche allein oder in Mischung vorhanden sein können. Der Rest Rₐ₁₄ kann ebenfalls der Alkylrest von Polykondensationsprodukten von hydroxyfunktionalisierten Säuren, beispielsweise Poly-12-hydroxystearinsäure oder Polyricinolsäure sein.

Weiterhin Gegenstand der Erfindung sind Verbindungen der Formel I, wobei n = o = p = 0, q = 1, W = Wasserstoff, m = 2 bis 4, bevorzugt 4, und Rₐ = Rₐ₁₄ ist. Wobei Rₐ₁₄ wie oben definiert ist.

Weiterhin Gegenstand der Erfindung sind Verbindungen der Formel I, wobei n = o = 0, W = Wasserstoff, m = p = q = 1, B = -CH₂-CH₂OH-CH₂-O-, und Rₐ = Rₐ₁₅ ist. Dabei ist Rₐ₁₅ der Alkylrest von Myristinsäure oder Kokosfettsäure.

Weiterhin Gegenstand der Erfindung sind Siloxanverbindungen der allgemeinen Formel III 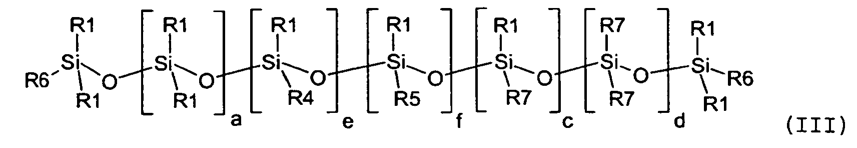 wobei
- N: a + c + d + e + f + 2 = 3 bis 850, vorzugsweise 6 bis 160,
- a: 1 bis 800, vorzugsweise 2 bis 150,
- c: 0 bis 10, vorzugsweise 0,
- d: 0 bis 10, vorzugsweise 0,
- e: 0 bis 400, vorzugsweise 2 bis 75,
- f: 0 bis 400, vorzugsweise 0 bis 75, ist,
dabei sind die Reste
- R1: unabhängig voneinander gleich oder verschieden und aus folgender Gruppe: gesättigte oder ungesättigte, gegebenenfalls verzweigte Alkylgruppen mit 1 bis 30 C-Atomen, Alkarylreste mit 7 bis 30 C-Atomen, Arylreste mit 6 bis 30 C-Atomen, vorzugsweise Alkylgruppen mit 1 bis 4 C-Atomen oder Phenyl, insbesondere Methyl,
- R4: unabhängig voneinander gleiche oder verschiedene Esterreste der allgemeinen Formel IIIa 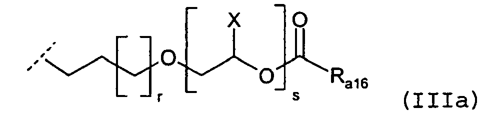 wobei
r 3 und
s 0 oder
r 1 und
s 1 bis 100, bevorzugt 1, ist,
X unabhängig voneinander gleiche oder verschiedene Reste aus der Gruppe -H, -CH₃, -CH₂CH₃ oder -Phenyl, bevorzugt Wasserstoff und
Rₐ₁₆ die Alkylreste einbasischer Fettsäuren auf Basis natürlicher pflanzlicher oder tierischer Öle mit 6 bis 30 Kohlenstoffatomen, insbesondere mit 8 bis 22 Kohlenstoffatomen, wie Capronsäure, Caprylsäure, Caprinsäure, Laurinsäure, Myristinsäure, Palmitinsäure, Palmitoleinsäure, Isostearinsäure, Stearinsäure, 12-Hydroxystearinsäure, Dihydroxystearinsäure, Ölsäure, Linolsäure, Petroselinsäure, Elaidinsäure, Arachinsäure, Behensäure, Erucasäure, Gadoleinsäure, Linolensäure, Eicosapentaensäure, Docosahexaensäure oder Arachidonsäure sind, welche allein oder in Mischung vorhanden sein können. Der Rest Rₐ₁₆ kann ebenfalls der Alkylrest von Polykondensationsprodukten von hydroxyfunktionalisierten Säuren, beispielsweise Poly-12-hydroxystearinsäure oder Polyricinolsäure sein,
weiterhin sind die Reste
- R5: unabhängig voneinander gesättigte oder ungesättigte, gegebenenfalls verzweigte Alkylgruppen mit 1 bis 30 C-Atomen oder Alkarylreste mit 7 bis 30 C-Atomen, vorzugsweise Alkylgruppen mit 6 bis 22 C-Atomen,
oder R⁵ die Reste der allgemeinen Formel IIIb 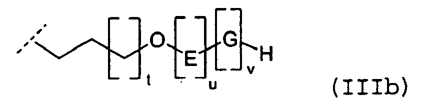 wobei
t 1 bis 28,
u 0 bis 100,
E ein Oxyalkenylrest der allg. Formel Ia 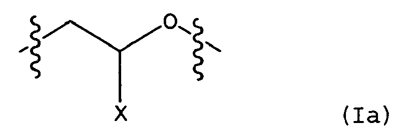 wobei
X unabhängig voneinander gleiche oder verschiedene Reste aus der Gruppe -H, -CH₃, -CH₂CH₃ oder -Phenyl, bevorzugt Wasserstoff, sind,
v 0 bis 20,
G für v = 1, ein vom Glycerin abgeleiteter Oxyalkenylrest der allg. Formel Ib 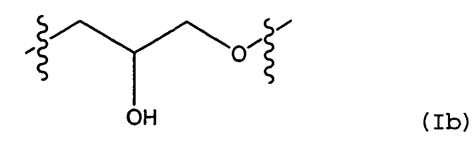 oder G der Rest der allg. Formel Ic ist, 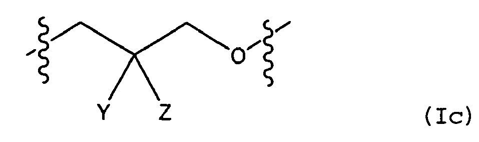
wobei
- Y und Z: unabhängig voneinander gleiche oder verschiedene Reste aus der Gruppe -H, -CH₃, -CH₂CH₃ oder -CH₂OH sind, oder
- G: für v ≥ 2, ein vom Polyglycerin abgleiteter Rest ist,
- R6: unabhängig voneinander R1, R4 oder R5, wobei R1, R4 und R5 wie oben definiert sind und wobei R6 = R4 ist, wenn e = 0, ist,
- R7: unabhängig voneinander gleiche oder verschiedene Reste der allgemeinen Formel IIIc 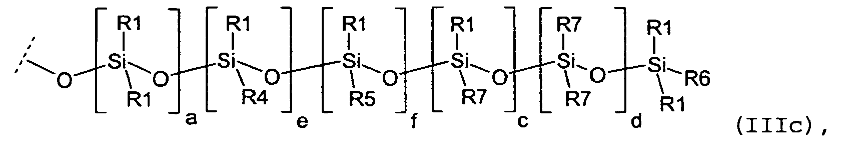 wobei
R1, R4, R5 und R6 wie oben definiert sind.

Reste gemäß der allgemeinen Formel IIIb können insbesondere Verbindungen mit
- t: 1 bis 28, insbesondere 1 und
- u: 0 bis 100, insbesondere 1 bis 20, besonders bevorzugt 1 und
- v: 0, oder
- t: 1 bis 28, insbesondere 1, und
- u: 0 und
- v: 1 bis 20, insbesondere 1 bis 6, besonders bevorzugt 1, sein.

Die folgenden Beispiele 1, 2, 11 und 12 sind nicht erfindungsgemäß sondern illustrieren den Stand der Technik. Die Beispiele 3 bis 10 und 13 bis 20 beschreiben das Verfahren zur Herstellung der erfindungsgemäßen Verbindungen sowie deren Eigenschaften näher. Die Beispiele dienen zur Illustration des Verfahrens und sollen in keiner Weise die Anwendungsbreite der Erfindung einschränken. Ergebnisse analytischer Nachweismethoden wie ¹H, ¹³C, ²⁹Si -NMR und GPC stehen in Einklang mit den angegebenen Strukturen der Produkte.

### Beispiele:

### Beispiel 1:

### PTSA-katalysierte Synthese von 1-Allylglyceryl-kokosfettsäureester

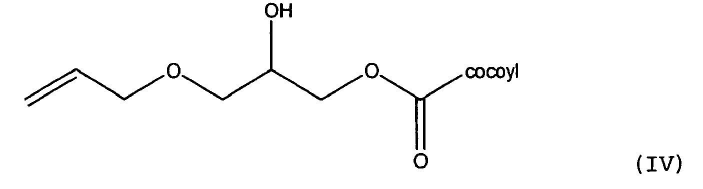

In einem Mehrhalsrundkolben werden 185,1 g 1-Allylglycerin und 213,4 g Kokosfettsäure vorgelegt und nach Zugabe von 0,4 g para-Toluensulfonsäure (PTSA) unter Stickstoffatmosphäre auf 170 °C erhitzt. Nach 7 Stunden wird die Reaktionsmischung abgekühlt, der Katalysator mit 1,51 g einer 50 %-igen K₂CO₃-Lösung neutralisiert, das Wasser im Vakuum abdestilliert und Feststoffe abfiltriert. Das Filtrat liefert 379 g Produkt ohne weitere Aufarbeitung als leicht braune Flüssigkeit.

### Beispiel 2:

### Zinn(II)oxalat katalysierte Synthese von 1-Allylglyceryl-kokosfettsäureester

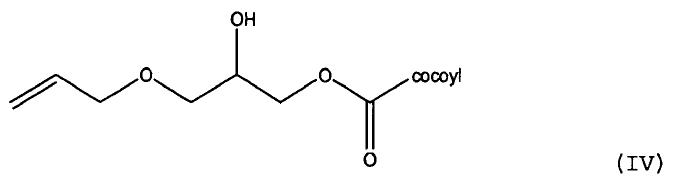

In einem Mehrhalsrundkolben werden 185,1 g 1-Allylglycerin und 213,4 g Kokosfettsäure vorgelegt und nach Zugabe von 0,7 g Zinn(II)oxalat auf 220 °C erhitzt. Nach 4 Stunden wird die Reaktionsmischung abgekühlt und der Katalysator mit Hilfe von 2,4 g Tinex P (Goldschmidt TIB, Mannheim) abfiltriert. Das Filtrat liefert 370 g Produkt ohne weitere Aufarbeitung als leicht braune Flüssigkeit.

### Beispiel 3:

### Enzymatische Synthese von 1-Allylglyceryl-kokosfettsäureester

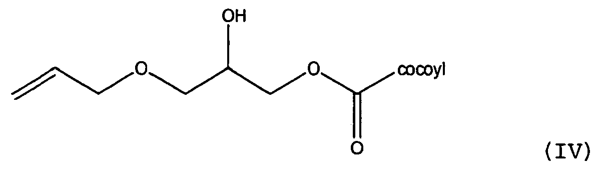

In einem Mehrhalsrundkolben werden 185,1 g 1-Allylglycerin und 213,4 g Kokosfettsäure vorgelegt und auf 50 °C erhitzt. Nach Zugabe von 19 g Novozym 435 (immobilisierte Lipase B aus *C. antarctica,* bezogen von Novozymes A/S, Bagsvaerd, Dänemark) wird Vakuum angelegt (20 mbar) und das Reaktionswasser abdestilliert. Nach 7 Stunden wird das immobilisierte Enzym abfiltriert. Das Filtrat liefert 379 g Produkt ohne weitere Aufarbeitung als farblose Flüssigkeit.

### Beispiel 4:

### Enzymatische Synthese von 1-Allylglyceryl-stearinsäureester

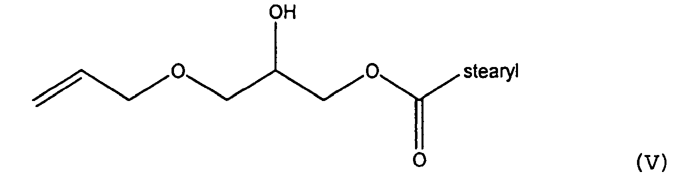

In einem Mehrhalsrundkolben werden 158,6 g 1-Allylglycerin und 237 g Stearinsäure vorgelegt und auf 60 °C erhitzt. Nach Zugabe von 19 g Lipozym RM IM (Immobilisierte Lipase aus *Mucor miehei,* Novozymes A/S) wird Vakuum angelegt (20 mbar) und das Reaktionswasser abdestilliert. Nach 48 Stunden wird das immobilisierte Enzym abfiltriert. Das Filtrat liefert 382 g Produkt ohne weitere Aufarbeitung als farblose Flüssigkeit.

### Beispiel 5:

### Enzymatische Synthese von Allyloxyethanol-kokosfettsäureester

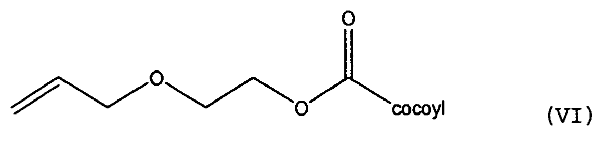

In einem Mehrhalsrundkolben werden 134,8 g Allyloxyethanol und 243,9 g Kokosfettsäure vorgelegt und auf 40 °C erhitzt. Nach Zugabe von 18 g Novozym 435 wird Vakuum angelegt (20 mbar) und das Reaktionswasser abdestilliert. Nach 10 Stunden wird das immobilisierte Enzym abfiltriert. Das Filtrat liefert 344 g Produkt ohne weitere Aufarbeitung als farblose Flüssigkeit.

### Beispiel 6:

### Enzymatische Synthese von Kokosfettsäurehexenylester

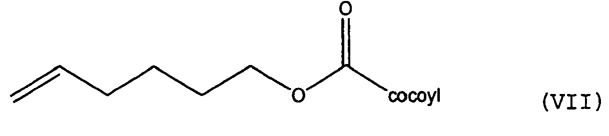

In einem Mehrhalsrundkolben werden 140,0 g Hex-5-en-1-ol und 258,1 g Kokosfettsäure vorgelegt und auf 60 °C erhitzt. Nach Zugabe von 19 g Novozym 435 wird Vakuum angelegt (20 mbar) und das Reaktionswasser abdestilliert. Nach 7 Stunden wird das immobilisierte Enzym abfiltriert. Das Filtrat liefert 360 g Produkt ohne weitere Aufarbeitung als farblose Flüssigkeit.

### Beispiel 7:

### Enzymatische Synthese von Undecylensäureoctylester

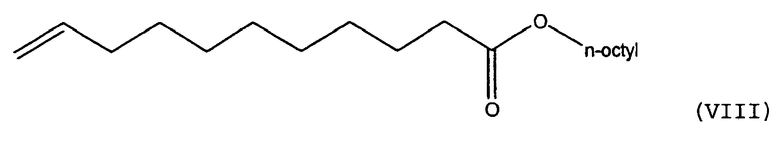

In einem Mehrhalsrundkolben werden 82 g 1-Octanol und 124,9 g Undecylensäuremethylester vorgelegt und auf 60 °C erhitzt. Nach Zugabe von 10 g Novozym 435 wird Vakuum angelegt (20 mbar) und das freiwerdende Methanol abdestilliert. Nach 6 Stunden wird das immobilisierte Enzym abfiltriert. Das Filtrat liefert 186 g Produkt ohne weitere Aufarbeitung als farblose Flüssigkeit.

### Beispiel 8:

### 2 eq. 1,6-Hexandioldiundecylenat

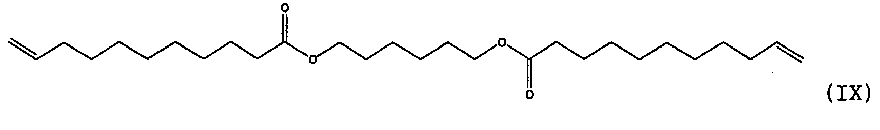

In einem Mehrhalsrundkolben werden 44,9 g 1,6-Hexandiol und 150,7 g Undecylensäuremethylester vorgelegt und auf 60 °C erhitzt. Nach Zugabe von 9,8 g Novozym 435 wird Vakuum angelegt (20 mbar) und das freiwerdende Methanol abdestilliert. Nach 6 Stunden wird das immobilisierte Enzym abfiltriert. Das Filtrat liefert 171 g Produkt ohne weitere Aufarbeitung als farblose Flüssigkeit.

### Beispiel 9:

### Adipinsäurediallyloxyethylester

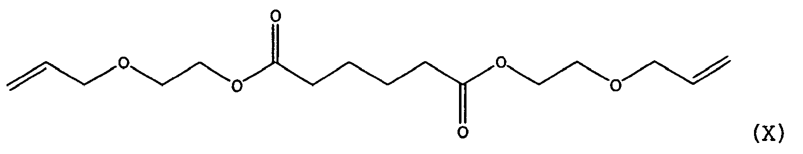

In einem Mehrhalsrundkolben werden 125,6 g Adipinsäure und 210,7 g Allyloxyethanol vorgelegt und auf 60 °C erhitzt. Nach Zugabe von 15,9 g Novozym 435 wird Vakuum angelegt (20 mbar) und das freiwerdende Wasser abdestilliert. Nach 8 Stunden wird das immobilisierte Enzym abfiltriert. Das Filtrat liefert 270 g Produkt ohne weitere Aufarbeitung als farblose Flüssigkeit.

### Beispiel 10:

### Allylpolyethylenglycol-7-laurat

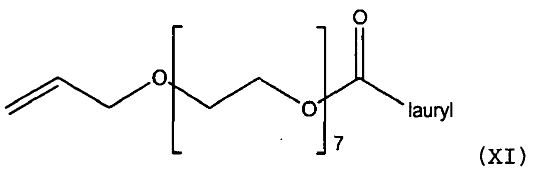

In einem Mehrhalsrundkolben werden 200,4 g Allylpolyethylenglycol-7 und 100,2 g Laurinsäure vorgelegt und auf 60 °C erhitzt. Nach Zugabe von 15 g Novozym 435 wird Vakuum angelegt (20 mbar) und das freiwerdende Wasser abdestilliert. Nach 8 Stunden wird das immobilisierte Enzym abfiltriert. Das Filtrat liefert 291 g Produkt ohne weitere Aufarbeitung als farblose Flüssigkeit.

### Beispiel 11:

### Versuch zur Hydrosilylierung des Reaktionsproduktes von Beispiel 1

Hergestellt werden soll ein Polysiloxan der allgemeinen Formel XII:

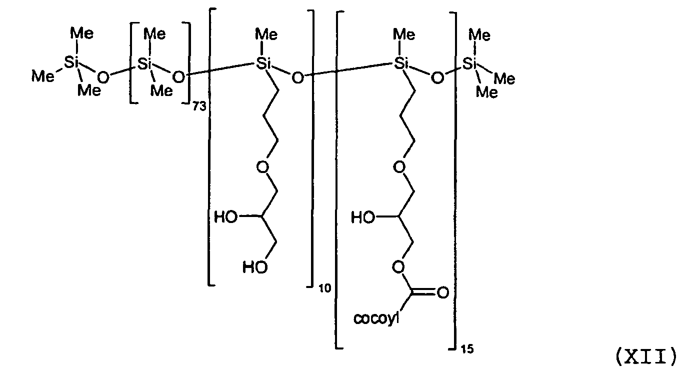

In einem Vierhalskolben, versehen mit Rührer, Tropftrichter, Thermometer und Rückflusskühler, werden 8,5 g (64 mmol) Glycerinmonoallylether, 31,5 g (96 mmol) des Glycerinmonoallyletherkokosfettsäureesters aus Beispiel 1 und 10 ppm Karstedt-Katalysator vorgelegt und auf 95 °C erhitzt. 34,1 g (123 mmol SiH) eines SiH-Siloxans der allgemeinen Formel XIII: 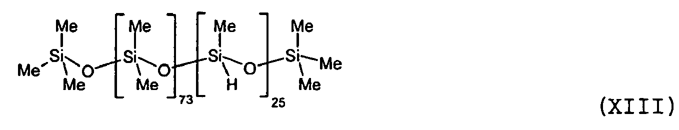 werden zugetropft und der Ansatz bei 95 °C gerührt. Der Ansatz vergelt sehr schnell, woraufhin die Reaktion abgebrochen wird.

### Beispiel 12:

### Versuch zur Hydrosilylierung des Reaktionsproduktes von Beispiel 2

Hergestellt werden soll ein Polysiloxan der allgemeinen Formel XII:

In einem Vierhalskolben, versehen mit Rührer, Tropftrichter, Thermometer und Rückflusskühler, werden 8,5 g (64 mmol) Glycerinmonoallylether, 31,5 g (96 mmol) des Glycerinmonoallyletherkokosfettsäureesters aus Beispiel 2 und 10 ppm Karstedt-Katalysator vorgelegt und auf 95 °C erhitzt. 34,1 g (123 mmol SiH) eines SiH-Siloxans der allgemeinen Formel XIII werden zugetropft und der Ansatz bei 95 °C gerührt. Der Ansatz vergelt sehr schnell, woraufhin die Reaktion abgebrochen wird.

### Beispiel 13:

### Hydrosilylierung des Reaktionsproduktes von Beispiel 3

Herstellung eines erfindungsgemäßen Polysiloxans der allgemeinen Formel XII:

In einem Vierhalskolben, versehen mit Rührer, Tropftrichter, Thermometer und Rückflusskühler, werden 8,5 g (64 mmol) Glycerinmonoallylether, 31,5 g (96 mmol) des Glycerinmonoallyletherkokosfettsäureesters aus Beispiel 3 und 10 ppm Karstedt-Katalysator vorgelegt und auf 95 °C erhitzt. 34,1 g (123 mmol SiH) eines SiH-Siloxans der allgemeinen Formel XIII werden zugetropft und der Ansatz für 1 h bei 95 °C gerührt. Laut SiH-Wert-Bestimmung wird ein vollständiger Umsatz des SiH-Siloxans erhalten. Flüchtige Anteile werden anschließend im Vakuum bei 110 °C abdestilliert. Es wird ein viskoses, leicht trübes, schwach gelbes Produkt erhalten.

### Beispiel 14:

### Hydrosilylierung des Reaktionsproduktes von Beispiel 4

Herstellung eines erfindungsgemäßen Polysiloxans der allgemeinen Formel XIV:

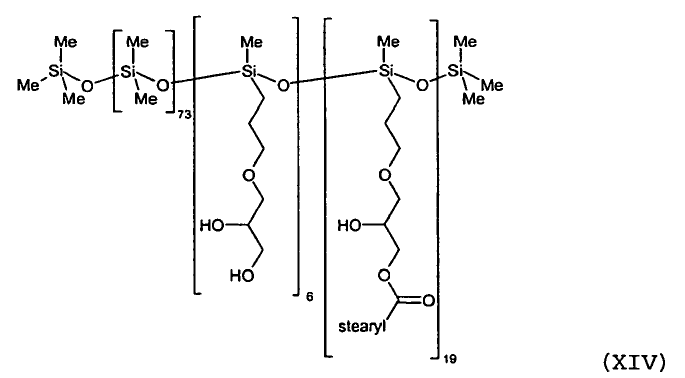

In einem Vierhalskolben, versehen mit Rührer, Tropftrichter, Thermometer und Rückflusskühler, werden 20,6 g (156 mmol) Glycerinmonoallylether, 203,9 g (494 mmol) des Glycerinmonoallyletherstearinsäureesters aus Beispiel 4 und 10 ppm Karstedt-Katalysator vorgelegt und auf 90 °C erhitzt. 142,5 g (500 mmol SiH) eines SiH-Siloxans der allgemeinen Formel XIII werden zugetropft und der Ansatz für 1,5 h bei 90 °C gerührt. Laut SiH-Wert-Bestimmung wird ein vollständiger Umsatz des SiH-Siloxans erhalten. Flüchtige Anteile werden anschließend im Vakuum bei 100 °C abdestilliert. Es wird ein hell-gelbes, wachsartig festes Produkt erhalten.

### Beispiel 15:

### Hydrosilylierung des Reaktionsproduktes von Beispiel 5

Herstellung eines erfindungsgemäßen Polysiloxans der allgemeinen Formel XV:

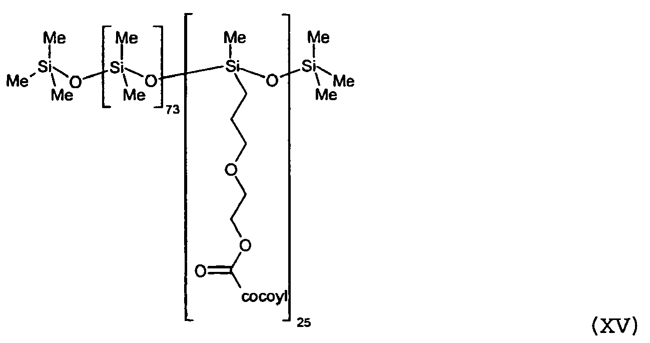

In einem Vierhalskolben, versehen mit Rührer, Tropftrichter, Thermometer und Rückflusskühler, werden 29,1 g (100 mmol) des Allyloxyethanolkokosfettsäureesters aus Beispiel 5 und 10 ppm Karstedt-Katalysator vorgelegt und auf 95 °C erhitzt. 22,2 g (77 mmol SiH) eines SiH-Siloxans der allgemeinen Formel XIII werden zugetropft und der Ansatz für 1 h bei 95 °C gerührt. Laut SiH-Wert-Bestimmung wird ein vollständiger Umsatz des SiH-Siloxans erhalten. Flüchtige Anteile werden anschließend im Vakuum bei 110 °C abdestilliert. Es wird ein leicht viskoses, schwach gelbes Produkt erhalten.

### Beispiel 16:

### Hydrosilylierung des Reaktionsproduktes von Beispiel 6

Herstellung eines erfindungsgemäßen Polysiloxans der allgemeinen Formel XVI:

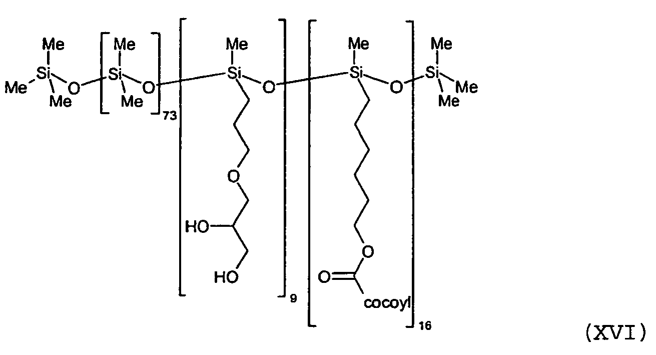

In einem Vierhalskolben, versehen mit Rührer, Tropftrichter, Thermometer und Rückflusskühler, werden 6,2 g (47 mmol) Glycerinmonoallylether, 23,6 g (83 mmol) des Kokosfettsäurehex-5-en-1-olesters aus Beispiel 6 und 10 ppm Karstedt-Katalysator vorgelegt und auf 95 °C erhitzt. 28,9 g (100 mmol SiH) eines SiH-Siloxans der allgemeinen Formel XIII werden zugetropft und der Ansatz für 1 h bei 95 °C gerührt. Laut SiH-Wert-Bestimmung wird ein vollständiger Umsatz des SiH-Siloxans erhalten. Flüchtige Anteile werden anschließend im Vakuum bei 110 °C abdestilliert. Es wird ein viskoses, leicht trübes, fast farbloses Produkt erhalten.

### Beispiel 17:

### Hydrosilylierung des Reaktionsproduktes von Beispiel 7

Herstellung eines erfindungsgemäßen Polysiloxans der allgemeinen Formel XVII:

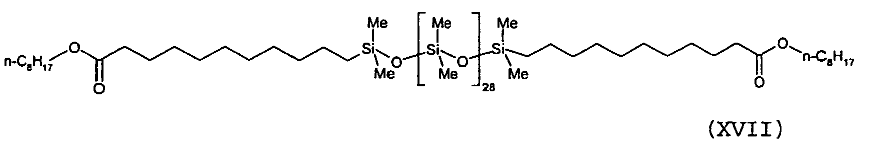

In einem Vierhalskolben, versehen mit Rührer, Tropftrichter, Thermometer und Rückflusskühler, werden 19,2 g (65 mmol) des Undecylensäureoctylesters aus Beispiel 7 und 10 ppm Karstedt-Katalysator vorgelegt und auf 95 °C erhitzt. 57,2 g (50 mmol SiH) eines SiH-Siloxans der allgemeinen Formel XVIII: 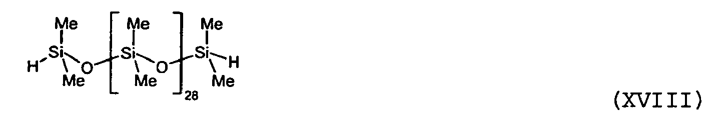 werden zugetropft und der Ansatz für 2 h bei 95 °C gerührt. Laut SiH-Wert-Bestimmung wird ein vollständiger Umsatz des SiH-Siloxans erhalten. Flüchtige Anteile werden anschließend im Vakuum bei 110 °C abdestilliert. Es wird eine klare, fast farblose Flüssigkeit erhalten.

### Beispiel 18:

### Hydrosilylierung des Reaktionsproduktes von Beispiel 8

Herstellung eines erfindungsgemäßen Polysiloxan-Copolymers der allgemeinen Formel XIX:

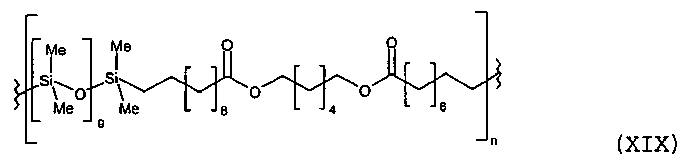

In einem Vierhalskolben, versehen mit Rührer, Tropftrichter, Thermometer und Rückflusskühler, werden 41,2 g (91 mmol) des 1,6-Hexandiol-undecylensäurediesters aus Beispiel 8 und 15 ppm cis-Platin-Katalysator vorgelegt und auf 120 °C erhitzt. 46,6 g (140 mmol SiH) eines SiH-Siloxans der allgemeinen Formel XX: 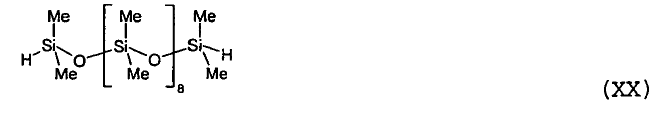 werden innerhalb von 10 min zugetropft und der Ansatz für 1 h bei 120 °C gerührt. Laut SiH-Wert-Bestimmung wird ein vollständiger Umsatz des SiH-Siloxans erhalten. Es wird ein leicht opakes, leicht gelbes Öl erhalten.
Molgewichtsverteilung laut GPC: M_{w} = 12319, Mₙ = 4672.

### Beispiel 19:

### Hydrosilylierung des Reaktionsproduktes von Beispiel 9

Herstellung eines erfindungsgemäßen Polysiloxan-Copolymers der allgemeinen Formel XXI:

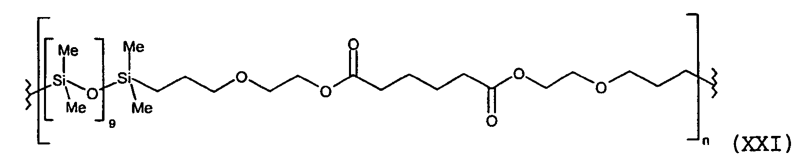

In einem Vierhalskolben, versehen mit Rührer, Tropftrichter, Thermometer und Rückflusskühler, werden 40,7 g (130 mmol) des Adipinsäure-allyloxyethanoldiesters aus Beispiel 9 und 5 ppm cis-Platin-Katalysator vorgelegt und auf 95 °C erhitzt. 66,6 g (200 mmol SiH) eines SiH-Siloxans der allgemeinen Formel XX werden innerhalb von 10 min zugetropft und der Ansatz für 1 h bei 95 °C gerührt. Laut SiH-Wert-Bestimmung wird ein vollständiger Umsatz des SiH-Siloxans erhalten. Es wird ein leicht opakes, leicht gelbes Öl erhalten.
Molgewichtsverteilung laut GPC: M_{w} = 8633, Mₙ = 3265.

### Beispiel 20:

### Hydrosilylierung des Reaktionsproduktes von Beispiel 10

Herstellung eines erfindungsgemäßen Polysiloxans der allgemeinen Formel XXII:

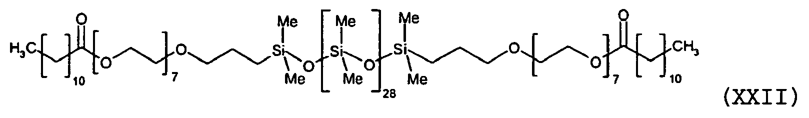

In einem Vierhalskolben, versehen mit Rührer, Tropftrichter, Thermometer und Rückflusskühler, werden 57,1 g (50 mmol SiH) eines SiH-Siloxans der allgemeinen Formel XVIII und 10 ppm Karstedt-Katalysator vorgelegt und auf 90 °C erhitzt. 38,4 g (130 mmol) des Allylpolyetherlaurats aus Beispiel 10 werden zugetropft und der Ansatz für 1 h bei 90 °C gerührt. Laut SiH-Wert-Bestimmung wird ein vollständiger Umsatz des SiH-Siloxans erhalten. Es wird eine leicht opake, fast farblose Flüssigkeit erhalten.

## Patentansprüche

1. Verfahren zur Herstellung von mit organischen Estern modifizierten Siloxanen durch Hydrosilylierung von Siloxanen mit terminal ungesättigten Estern, **dadurch gekennzeichnet, dass** die eingesetzten terminal ungesättigten Ester unter Verwendung mindestens eines Enzyms als Katalysator hergestellt werden.

2. Verfahren nach Anspruch 1, **dadurch gekennzeichnet, dass** als terminal ungesättigte Ester Verbindungen der allgemeinen Formel I 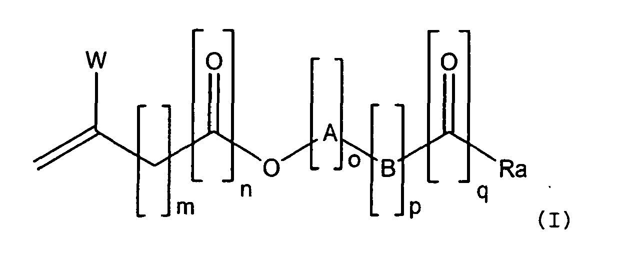 verwendet werden, wobei
W Wasserstoff oder Methyl, bevorzugt Wasserstoff,
m 0 bis 28, bevorzugt 1 bis 17,
n 0 oder 1,
o 0 bis 100, ist,
A ein Oxyalkenylrest der allg. Formel Ia 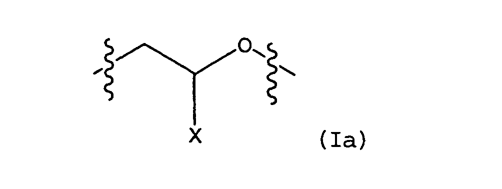
ist, wobei
X unabhängig voneinander gleiche oder verschiedene Reste aus der Gruppe -H, -CH₃ , -CH₂CH₃ oder -Phenyl, bevorzugt Wasserstoff, ist,
p 0 bis 20,
B für p = 1, ein von Glycerin abgeleiteter, gegebenen- falls mit R_{b} veresterter, Oxyalkenylrest der allg. For- mel Ib ist, 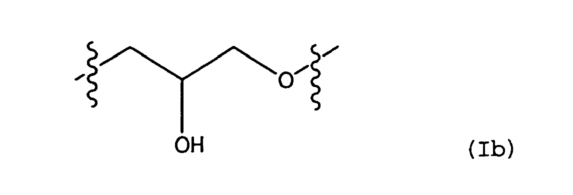
wobei
R_{b} der Acylrest linearer, verzweigter, gesättigter oder ungesättigter, gegebenenfalls zusätzliche Hydroxygruppen tragender Carbonäuren mit 2 bis 30 C-Atomen ist, oder
B für p = 1, der Rest der allg. Formel Ic ist, 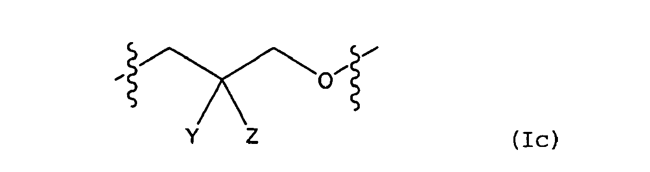
wobei
Y und Z unabhängig voneinander gleiche oder verschiedene Reste aus der Gruppe -H, -CH₃, -CH₂CH₃, -CH₂OH oder -CH₂OR_{b} sind, wobei
R_{b} wie oben definiert ist, oder
B für p ≥ 2, ein vom Polyglycerin abgleiteter, gege- benenfalls mit R_{b} veresterter, Rest ist, wobei
R_{b} wie oben definiert ist,
Rₐ Wasserstoff, Rₐ₁ oder Rₐ₂ ist, wobei
Rₐ₁ ein linearer oder verzweigter, gesättigter oder ungesättigter, gegebenenfalls Hydroxygruppen tra- gender, gegebenenfalls mit Carbonsäuren veresterte Hydroxygruppen tragender, gegebenenfalls Amino-, Alkylamino- oder Dialkylaminogruppen tragender Alkylrest mit 1 bis 200 C-Atomen,
Rₐ₂ der Rest der allgemeinen Formel Id ist 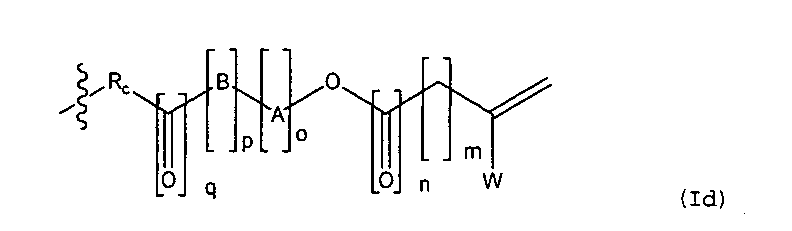
und
R_{c} ein gesättigter oder ungesättigter difunktioneller Kohlenwasserstoffrest mit 2 bis 20 C-Atomen ist,
wobei
n + q = 1, ist und wenn
Rₐ Wasserstoff ist
q 0 und
o + p ≥ 1, ist.

3. Verfahren nach Anspruch 2, **dadurch gekennzeichnet, dass** Ester der allgemeinen Formel I verwendet werden,
wobei
n,o,p 0,
W Wasserstoff,
m 1 bis 28, bevorzugt 1 bis 4,
q 1,
Rₐ, Rₐ₁ = Rₐ₁₁ oder Rₐ = Rₐ₂ ist, wobei
Rₐ₂ wie in Anspruch 2 definiert ist, und
Rₐ₁₁ den von handelsüblichen Säuren, wie beispielsweise Es- sigsäure, Propansäure, Butansäure, Pentansäure, Chloressigsäure, Trifluoressigsäure, Ethylhexansäure, Isononansäure, Isotridecansäure oder Isostearinsäure abgeleiteten Alkylrest oder den von einbasischen Fett- säuren auf Basis natürlicher pflanzlicher oder tieri- scher Öle mit 6 bis 30 Kohlenstoffatomen, insbesondere mit 8 bis 22 Kohlenstoffatomen, wie Capronsäure, Caprylsäure, Caprinsäure, Laurinsäure, Myristinsäure, Palmitinsäure, Palmitoleinsäure, Isostearinsäure, Stearinsäure, 12-Hydroxystearinsäure, Dihydroxystea- rinsäure, Ölsäure, Linolsäure, Petroselinsäure, Elaidinsäure, Arachinsäure, Behensäure, Erucasäure, Gadoleinsäure, Linolensäure, Eicosapentaensäure, Do- cosahexaensäure, Arachidonsäure, welche allein oder in Mischung eingesetzt werden können, abgeleiteten Alkylrest oder den von Polykondensationsprodukten von hydroxyfunktionalisierten Säuren, beispielsweise Poly- 12-hydroxystearinsäure oder Polyricinolsäure, abgeleiteten Alkylrest darstellt.

4. Verfahren nach Anspruch 2, **dadurch gekennzeichnet, dass** Ester der allgemeinen Formel I verwendet werden,
wobei
n,p 0,
W Wasserstoff,
m 1 bis 28, bevorzugt 1 bis 4, insbesondere 1,
o 1 bis 100, insbesondere 1,
q 1, ist,
Rₐ Rₐ₁ = Rₐ₁₁ oder Rₐ = Rₐ₂ ist, wobei Rₐ₁₁ wie in Anspruch 3 definiert ist.

5. Verfahren nach Anspruch 2, **dadurch gekennzeichnet, dass** Ester der allgemeinen Formel I verwendet werden,
wobei
n,o 0,
W Wasserstoff,
m 1 bis 28, bevorzugt 1 bis 4, insbesondere 1,
B -CH₂-CH₂OH-CH₂-O- oder -CH₂-CH₂OR_{b}-CH₂-O-, wobei R_{b} wie in Anspruch 2 definiert ist,
p 1 bis 20, bevorzugt 1,
q 1, ist,
Rₐ Rₐ₁ = Rₐ₁₁ oder Rₐ = Rₐ₂ ist, wobei Rₐ₁₁ wie in Anspruch 3 definiert ist.

6. Verfahren nach Anspruch 2, **dadurch gekennzeichnet, dass** Ester der allgemeinen Formel I verwendet werden,
wobei
n, o 0,
W Wasserstoff,
m 1 bis 28, bevorzugt 1 bis 4, insbesondere 1,
B -CH₂-CH₂YZ-CH₂-O-,
P q = 1,
Rₐ Rₐ₁ = Rₐ₁₁ oder Rₐ = Rₐ₂,
wobei
Rₐ₁₁ wie in Anspruch 3 definiert ist.

7. Verfahren nach Anspruch 2, **dadurch gekennzeichnet, dass** Ester der allgemeinen Formel I verwendet werden,
wobei
n 1,
W Wasserstoff oder CH₃, bevorzugt Wasserstoff,
m 0 bis 27, bevorzugt 1 bis 10,
o,p 0,
Rₐ Rₐ₁ = Rₐ₁₂ oder Rₐ = Rₐ₂, ist,
wobei
Rₐ₁₂ der Kohlenwasserstoffrest eines substituierten oder un- substituierten, gegebenenfalls verzweigten, gegeben- falls eine oder mehrere Mehrfachbindung(en) enthalten- den, gegebenenfalls Hydroxygruppen, gegebenfalls mit Carbonsäuren veresterte Hydroxygruppen, gegebenfalls Amino-, Alkylamino- oder Dialkylaminogruppen tragenden Alkohols mit 2 bis 30 C-Atomen, bevorzugt mit 6 bis 22 C-Atomen, ist.

8. Verfahren nach einem der Ansprüche 1 bis 7, **dadurch gekennzeichnet, dass** zur Herstellung der Ester statt der Säuren die entsprechenden Ester leichtflüchtiger Alkohole zu einer Umesterung eingesetzt werden, bevorzugt Methyl-, Ethyl- und Vinylester.

9. Verfahren nach einem der Ansprüche 1 bis 8, **dadurch gekennzeichnet, dass** Enzyme aus der Gruppe der hydrolytischen Enzyme, z. B. Lipasen, Esterasen oder Proteasen, vorzugsweise Lipasen, besonders bevorzugt Lipase B aus Candida antarctica, verwendet werden.

10. Verfahren nach einem der Ansprüche 1 bis 9, **dadurch gekennzeichnet, dass** als SiH-Polysiloxan eine Verbindung der allgemeinen Formel II 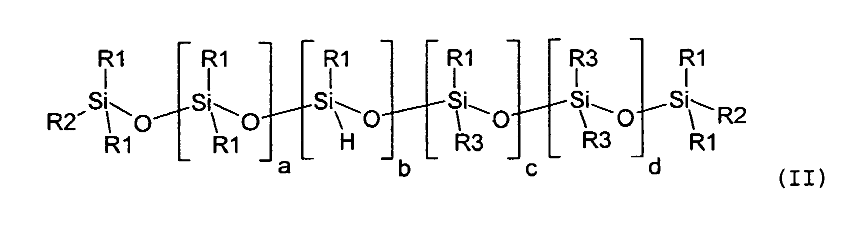 eingesetzt wird, wobei
N a + b + c + d + 2 = 3 bis 850, vorzugsweise 6 bis 160,
a 1 bis 800, vorzugsweise 2 bis 150,
b 0 bis 400, vorzugsweise 2 bis 75,
c 0 bis 10, vorzugsweise 0,
d 0 bis 10, vorzugsweise 0, ist, und
worin die Reste
R1 unabhängig voneinander gleich oder verschieden und aus folgender Gruppe sind, gesättigte oder ungesättigte, gegebenenfalls verzweigte Alkylgruppen mit 1 bis 30 C-
R2 Atomen, Alkarylreste mit 7 bis 30 C-Atomen, Arylreste mit 6 bis 30 C-Atomen, vorzugsweise Alkylgruppen mit 1 bis 4 C-Atomen oder Phenyl, insbesondere Methyl, unabhängig voneinander Wasserstoff oder R1,
R3 unabhängig voneinander gleiche oder verschiedene Reste der allgemeinen Formel IIa, sind.

11. Verbindungen der allgemeinen Formel I, **dadurch gekennzeichnet, dass** n = p = 0, W = Wasserstoff, X = Wasserstoff oder Methyl, bevorzugt Wasserstoff, m = q = 1, o = 2 bis 100, bevorzugt 3 bis 100, und Rₐ = Rₐ₁₄ ist, wobei Rₐ₁₄ der Alkylrest von handelsüblichen Säuren mit 4 bis 30 Kohlenstoffatomen oder von einbasischen Fettsäuren auf Basis natürlicher pflanzlicher oder tierischer Öle mit 6 bis 30 Kohlenstoffatomen, welche allein oder in Mischung vorhanden sein können, oder von Polykondensationsprodukten von hydroxyfunktionalisierten Säuren ist.

12. Verbindungen der allgemeinen Formel I, **dadurch gekennzeichnet, dass** n = o = 0, W = Wasserstoff, m = p = q = 1, B = -CH₂-CH₂OH-CH₂-O-, und Rₐ = Rₐ₁₅ ist, wobei Rₐ₁₅ der Alkylrest von Myristinsäure oder Kokosfettsäure ist.

13. Verbindungen der allgemeinen Formel III, 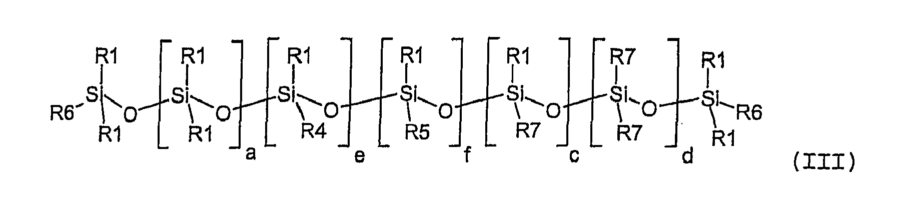 **dadurch gekennzeichnet, dass**
N a + c + d + e + f + 2 = 3 bis 850, vorzugsweise 6 bis 160,
a 1 bis 800, vorzugsweise 2 bis 150,
c 0 bis 10, vorzugsweise 0,
d 0 bis 10, vorzugsweise 0,
e 0 bis 400, vorzugsweise 2 bis 75 und
f 0 bis 400, vorzugsweise 0 bis 75 ist, sowie die Reste
R1 unabhängig voneinander gleich oder verschieden und aus der Gruppe der gesättigten oder ungesättigten, gegebenenfalls verzweigten Alkylgruppen mit 1 bis 30 C-Atomen, Alkarylreste mit 7 bis 30 C-Atomen, Arylreste mit 6 bis 30 C-Atomen, vorzugsweise Alkylgruppen mit 1 bis 4 C-Atomen oder Phenyl, sind,
R4 unabhängig voneinander gleiche oder verschiedene Esterreste der allgemeinen Formel IIIa 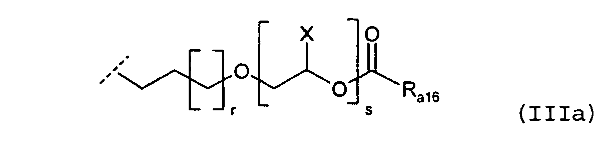
mit
r 1 und
s 1 oder
r 3 und s 0 und
X unabhängig voneinander gleiche oder verschiedene Reste aus der Gruppe -H, -CH₃, -CH₂CH₃ oder -Phenyl, bevorzugt Wasserstoff, sind,
Rₐ₁₆ die Alkylreste einbasischer Fettsäuren auf Basis natürlicher pflanzlicher oder tierischer Öle mit 6 bis 30 Kohlenstoffatomen, insbesondere mit 8 bis 22 Kohlenstoffatomen, sind,
R5 unabhängig voneinander gesättigte oder ungesättigte, gegebenenfalls verzweigte Alkylgruppen mit 1 bis 30 C- Atomen oder Alkarylreste mit 7 bis 30 C-Atomen, vor- zugsweise Alkylgruppen mit 6 bis 22 C-Atomen oder R5 die Reste der allgemeinen Formel IIIb sind 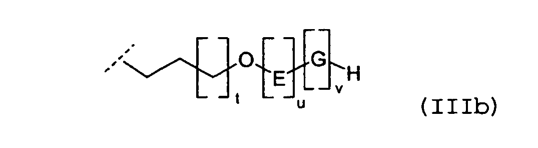
mit
t 1 bis 28,
u 0 bis 100,
E ein Oxyalkenylrest der allg. Formel Ia,
v 0 bis 20,
G für v = 1, ein vom Glycerin abgeleiteter Oxy- alkenylrest der allg. Formel Ib oder G für v = 1 der Rest der allg. Formel Ic mit Y und Z unabhängig voneinander gleiche oder verschiedene Reste aus der Gruppe -H, -CH₃, -CH₂CH₃ oder -CH₂OH sind, oder G für v ≥ 2 ein von Polyglycerin abglei- teter Rest ist,
R6 unabhängig voneinander R1, R4 oder R5 sind, wobei R1, R4 und R5 wie oben definiert sind und R6 gleich R4 ist, wenn e = 0 ist,
R7 unabhängig voneinander gleiche oder verschiedene Reste der allgemeinen Formel IIIc sind, 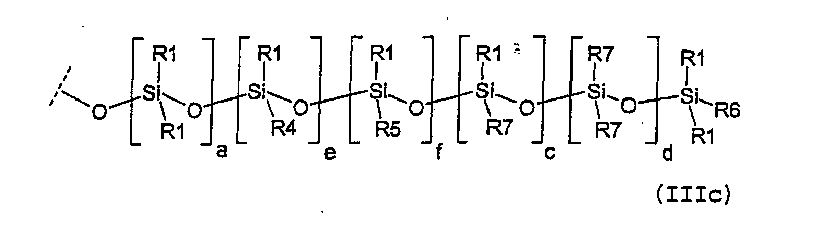 wobei
R1, R4, R5 und R6 wie oben definiert sind.

14. Verbindungen gemäß Anspruch 13, **dadurch gekennzeichnet, dass**
t 1 bis 28, insbesondere 1 und
u 1 bis 100, insbesondere 1 bis 20, besonders bevorzugt 1 und
v 0,
ist.

15. Verbindungen gemäß Anspruch 13, **dadurch gekennzeichnet, dass**
t 1 bis 28, insbesondere 1 und
u 0 und
v 1 bis 20, insbesondere 1 bis 6, besonders bevorzugt 1, ist.

16. Verbindungen der allgemeinen Formel III, 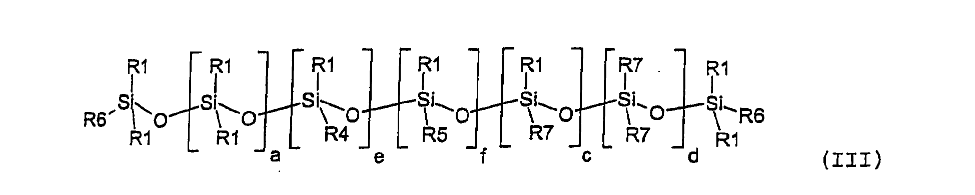 **dadurch gekennzeichnet, dass**
N a + c + d + e + f + 2 = 3 bis 850, vorzugsweise 6 bis 160,
a 1 bis 800, vorzugsweise 2 bis 150,
c 0 bis 10, vorzugsweise 0,
d 0 bis 10, vorzugsweise 0 und
e 0 bis 400, vorzugsweise 2 bis 75,
f 0, sowie die Reste
R1 unabhängig voneinander gleich oder verschieden und aus der Gruppe der gesättigten oder ungesättigten, gegebenenfalls verzweigten Alkylgruppen mit 1 bis 30 C-Atomen, Alkarylreste mit 7 bis 30 C-Atomen, Arylreste mit 6 bis 30 C-Atomen, vorzugsweise Alkylgruppen mit 1 bis 4 C-Atomen oder Phenyl, sind,
R4 unabhängig voneinander gleiche oder verschiedene Esterreste der allgemeinen Formel IIIa 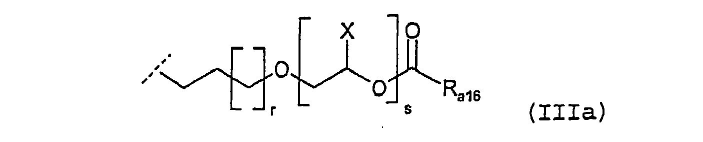 mit
r 1 und
s 2 bis 100, bevorzugt 3 bis 80 und
X unabhängig voneinander gleiche oder verschiedene Reste aus der Gruppe -H, -CH₃, -CH₂CH₃ oder -Phenyl, bevorzugt Wasserstoff oder -CH₃, besonders bevorzugt Wasserstoff, sind,
Rₐ₁₆ die Alkylreste einbasischer Fettsäuren auf Basis natürlicher pflanzlicher oder tierischer Öle mit 6 bis 30 Kohlenstoffatomen, insbesondere mit 8 mit 22 Kohlenstoffatomen, sind,
R6 unabhängig voneinander R1 oder R4 sind, wobei R1 und R4 wie oben definiert sind und R6 gleich R4 ist, wenn e = 0 ist,
R7 der unabhängig voneinander gleiche oder verschiedene Reste allgemeinen Formel IIIc sind, 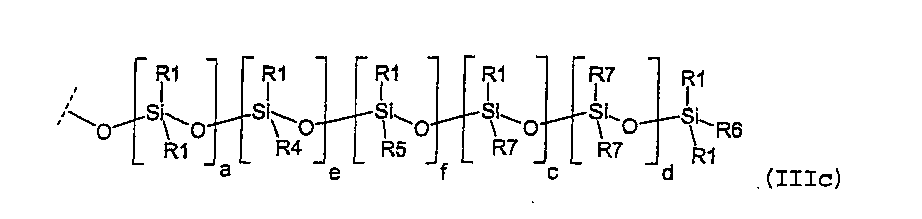 wobei R1, R4, R5 und R6 wie oben definiert sind.

17. Verbindungen, die erhalten werden durch die Hydrosilylierung von Estern der allgemeinen Formel I, 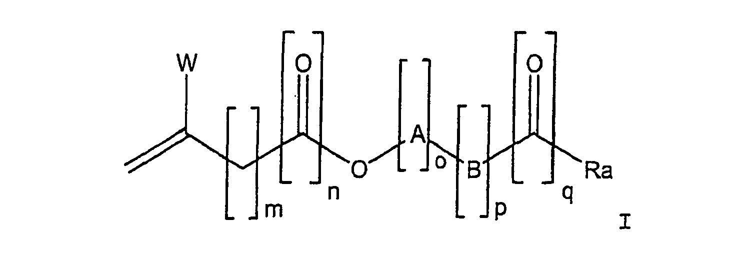 **dadurch gekennzeichnet, dass**
W Wasserstoff oder Methyl,
m 0 bis 28, bevorzugt 1 bis 17,
n 0 oder 1,
A Oxyalkenylrest der allg. Formel Ia 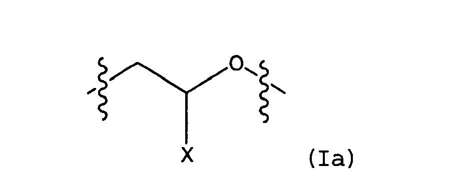
wobei
o X unabhängig voneinander gleiche oder verschiedene Reste aus der Gruppe -H, -CH₃, -CH₂CH₃ oder -Phenyl, bevorzugt Wasserstoff, ist, 0 bis 100,
p 0,
q 0 oder 1,
n + q 1,
Rₐ = Rₐ₂ oder Rₐ₃,
Rₐ₂ Rest der allgemeinen Formel Id, 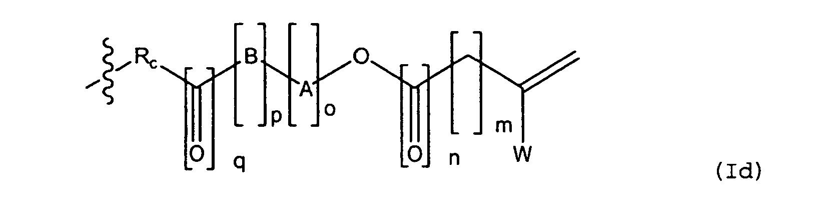
R_{c} gesättigter oder ungesättigter difunktioneller Kohlen- wasserstoffrest mit 2 bis 20 C-Atomen,
Rₐ₃ linearer oder verzweigter terminal ungesättigter Alkylrest mit 2 bis 30 C-Atomen, bevorzugt 3 bis 30 C- Atomen, der gegebenenfalls Hydroxygruppen und/oder mit Carbonsäuren veresterte Hydroxygruppen und/oder weitere Mehrfachbindungen trägt,
mit Siloxanen der allgemeinen Formel V, 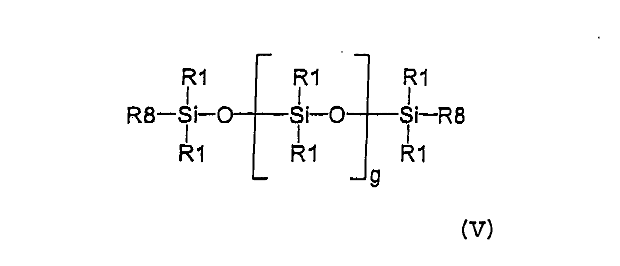 wobei
g 3 bis 400,
R1 unabhängig voneinander gleich oder verschieden und aus der Gruppe der gesättigten oder ungesättigten, gege- benenfalls verzweigten Alkylgruppen mit 1 bis 30 C- Atomen, Alkarylreste mit 7 bis 30 C-Atomen, Arylreste mit 6 bis 30 C-Atomen,
R8 unabhängig voneinander R1 oder Wasserstoff, wobei gilt, dass im Mittel mehr Reste Wasserstoff sind,
in Gegenwart eines hydrosilylierungsaktiven Katalysators, wobei gilt, dass das molare Verhältnis der SiH-Funktionen des Siloxanes bezogen auf die Doppelbindungen des Esters 0,8 bis 1,2 beträgt.

18. Verbindungen gemäß Anspruch 17, **dadurch gekennzeichnet,**
**dass**
W Wasserstoff,
m 0 bis 28, bevorzugt 1 bis 17,
n 1,
o 0,
p 0,
q 0,
Rₐ Rₐ₂,
ist.

19. Verbindungen gemäß Anspruch 17, **dadurch gekennzeichnet,**
**dass**
W Wasserstoff,
m 0 bis 28, bevorzugt 1 bis 17,
n 0,
o 0,
p 0,
q 1,
Rₐ Rₐ₂,
ist.

20. Verbindungen gemäß Anspruch 17, **dadurch gekennzeichnet,**
**dass**
W Wasserstoff,
m 0 bis 28, bevorzugt 1 bis 17,
n 0,
o 1 bis 100,
X Wasserstoff oder Methyl, bevorzugt Wasserstoff,
p 0,
q 1,
Rₐ Rₐ₂,
ist.

21. Verbindungen gemäß Anspruch 17, **dadurch gekennzeichnet, dass**
W Wasserstoff,
m 0 bis 28, bevorzugt 1 bis 17, besonders bevorzugt 1,
n 0,
o 1 bis 100, bevorzugt 3 bis 100,
X Wasserstoff oder Methyl, bevorzugt Wasserstoff,
p 0,
q 1,
Rₐ Rₐ₃,
ist.

## Claims

1. Process for preparing siloxanes modified with organic esters, by hydrosilylating siloxanes with terminally unsaturated esters, **characterized in that** the terminally unsaturated esters used are prepared using at least one enzyme as catalyst.

2. Process according to Claim 1, **characterized in that** terminally unsaturated esters used are compounds of the general formula I 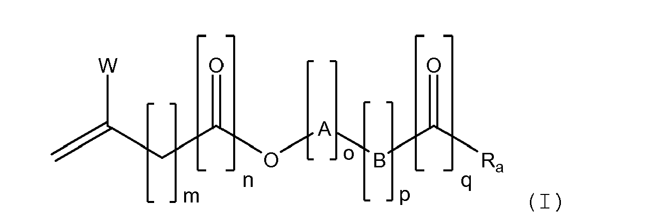 where
W is hydrogen or methyl, preferably hydrogen,
m is 0 to 28, preferably 1 to 17,
n is 0 or 1,
o is 0 to 100,
A is an oxyalkenyl radical of the general formula Ia 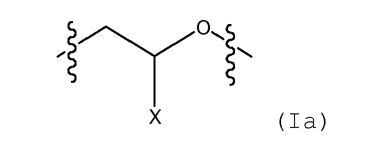
where
X radicals independently are identical or different radicals from the group -H, -CH₃, -CH₂CH₃ or -phenyl, preferably hydrogen,
p is from 0 to 20,
B if p = 1 is a glycerol-derived oxyalkenyl radical, optionally esterified with R_{b}, of the general formula Ib 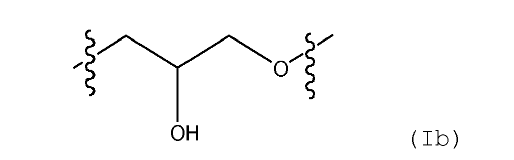
where
R_{b} is the acyl radical of linear, branched, saturated or unsaturated carboxylic acids having 2 to 30 carbon atoms and optionally carrying additional hydroxyl groups, or
B if p = 1 is the radical of the general formula Ic 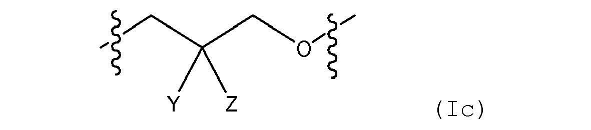
where
Y and Z independently of one another are identical or different radicals from the group -H, -CH₃, -CH₂CH₃, -CH₂OH or -CH₂OR_{b}, where
R_{b} is is defined as above, or
B if p ≥ 2 is a polyglycerol-derived radical optionally esterified with R_{b},
where
R_{b} is defined as above,
Rₐ is hydrogen, Rₐ₁ or Rₐ₂, where
Rₐ₁ is a linear or branched, saturated or unsaturated alkyl radical having 1 to 200 carbon atoms which optionally carries hydroxyl groups, optionally carries hydroxyl groups esterified with carboxylic
Rₐ₂ acids and optionally carries amino, alkylamino or dialkylamino groups, is a radical of the general formula Id 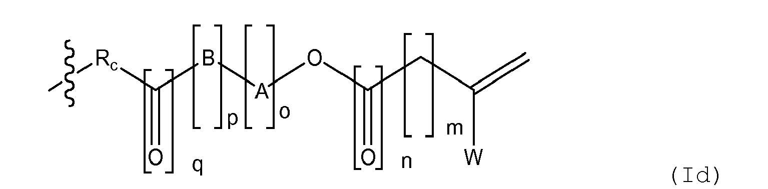 and
R_{c} is a saturated or unsaturated difunctional hydrocarbon radical having 2 to 20 carbon atoms,
where
n + q = 1, and if
Rₐ is hydrogen,
q is 0 and
o + p ≥ 1.

3. Process according to Claim 2, **characterized in that** esters of the general formula I are used where
n, o and p are 0,
W is hydrogen,
m is 1 to 28, preferably 1 to 4,
q is 1,
Rₐ, Rₐ₁ - Rₐ₁₁ or Rₐ = Ra₂,
where
Rₐ₂ is defined as in claim 2 and
Rₐ₁₁ is the alkyl radical derived from commercially customary acids, such as acetic acid, propanoic acid, butanoic acid, pentanoic acid, chloroacetic acid, trifluoroacetic acid, ethylhexanoic acid, isononanoic acid, isotridecanoic acid or isostearic acid, or the alkyl radical derived from mono-basic fatty acids based on natural vegetable or animal oils having 6 to 30 carbon atoms, in particular having 8 to 22 carbon atoms, such as caproic acid, caprylic acid, capric acid, lauric acid, myristic acid, palmitic acid, palmitoleic acid, isostearic acid, stearic acid, 12- hydroxystearic acid, dihydroxystearic acid, oleic acid, linoleic acid, petroselenic acid, elaidic acid, arachidic acid, behenic acid, erucic acid, gadoleic acid, linolenic acid, eicosapentaenoic acid, docosahexaenoic acid, arachidonic acid, which can be used alone or in a mixture, or the alkyl radical derived from polycondensation products of hydroxy- functionalized acids, such as poly-12-hydroxy- stearic acid or polyricinoleic acid.

4. Process according to Claim 2, **characterized in that** esters of the general formula I are used
where
W n and p are 0, is hydrogen,
m is 1 to 28, preferably 1 to 4, in particular 1,
o is 1 to 100, in particular 1,
q is 1,
Rₐ is Rₐ₁ = Rₐ₁₁ or Rₐ = Rₐ₂, where Rₐ₁₁ is defined as in Claim 3.

5. Process according to Claim 2, **characterized in that** esters of the general formula I are used
where
W n and o are 0, is hydrogen,
m is 1 to 28, preferably 1 to 4, in particular 1,
B is -CH₂-CH₂OH-CH₂-O- or -CH₂-CH₂OR_{b}-CH₂-O-, where R_{b} is defined as in claim 2,
p is 1 to 20, preferably 1,
q is 1,
Rₐ is Rₐ₁ = Rₐ₁₁ or Rₐ = Rₐ₂, where Rₐ₁₁ is defined as in Claim 3.

6. Process according to Claim 2, **characterized in that** esters of the general formula I are used where
W n and o are 0, is hydrogen,
m is 1 to 28, preferably 1 to 4, in particular 1,
B is -CH₂-CYZ-CH₂-O-, where Y and Z are defined as in Claim 2,
p is q = 1,
Rₐ is Rₐ₁ = Rₐ₁₁ or Rₐ = Rₐ₂, where
Rₐ₁₁ is defined as in Claim 3.

7. Process according to Claim 2, **characterized in that** esters of the general formula I are used
where
n is 1,
W is hydrogen or CH₃, preferably hydrogen,
m is 0 to 27, preferably 1 to 10,
o and p are 0,
Rₐ is Rₐ₁ = Rₐ₁₂ or Rₐ = Rₐ₂,
where
Rₐ₁₂ is the hydrocarbon radical of a substituted or unsubstituted, optionally branched alcohol having 2 to 30 carbon atoms, preferably having 6 to 22 carbon atoms, which optionally contains one or more multiple bonds, optionally carries hydroxyl groups, optionally carries hydroxyl groups esterified with carboxylic acids or optionally carries amino, alkylamino or dialkylamino groups.

8. Process according to any one of Claims 1 to 7, **characterized in that** the esters are prepared using rather than the acids the corresponding esters of volatile alcohols for a transesterification, preferably methyl, ethyl, and vinyl esters.

9. Process according to any one of Claims 1 to 8, **characterized in that** enzymes from the group of hydrolytic enzymes are used, examples being lipases, esterases or proteases, preferably lipases, more preferably lipase B from *Candida antarctica.*

10. Process according to any one of Claims 1 to 9, **characterized in that** as SiH-polysiloxane a compound of the general formula II 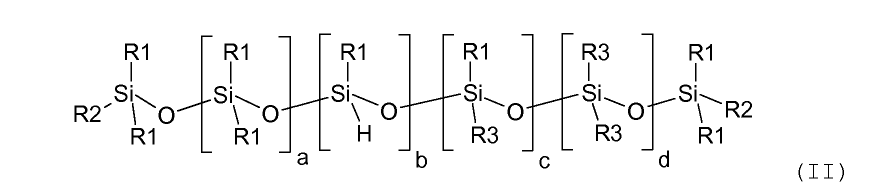 is used where
N a + b + c + d + 2 = 3 to 850, preferably 6 to 160,
a is 1 to 800, preferably 2 to 150,
b is 0 to 400, preferably 2 to 75,
c is 0 to 10, preferably 0,
d is 0 to 10, preferably 0, and
in which the radicals
R1 independently of one another are identical or different and from the following group: saturated or unsaturated, optionally branched alkyl groups having 1 to 30 carbon atoms, alkaryl radicals having 7 to 30 carbon atoms, aryl radicals having 6 to 30 carbon atoms, preferably alkyl groups having 1 to 4 carbon atoms or phenyl, especially methyl,
R2 independently of one another are hydrogen or R1,
R3 independently of one another are identical or different radicals of the general formula IIa.

11. Compound of the general formula I **characterized in that** n = p = 0, W = hydrogen, X = hydrogen or methyl, preferably hydrogen, m = q = 1, o = 2 to 100, preferably 3 to 100, and Rₐ = Rₐ₁₄, where Rₐ₁₄ is the alkyl radical of commercially customary acids having 4 to 30 carbon atoms or of mono-basic fatty acids based on natural vegetable or animal oils having 6 to 30 carbon atoms, which may be present alone or in a mixture, or of polycondensation products of hydroxy-functionalized acids.

12. Compound of the general formula I **characterized in that** n = o = 0, W = hydrogen, m = p = q = 1, B = -CH₂-CH₂OH-CH₂-O-, and Rₐ = Rₐ₁₅, where Rₐ₁₅ is the alkyl radical of myristic acid or coconut fatty acid.

13. Compound of the general formula III, 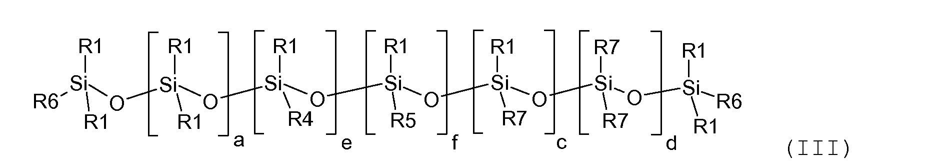 **characterized in that**
Na + b + c + d + e+ f + 2 = 3 to 850, preferably 6 to 160,
a is 1 to 800, preferably 2 to 150,
c is 0 to 10, preferably 0,
d is 0 to 10, preferably 0,
e is 0 to 400, preferably 2 to 75, and
f is 0 to 400, preferably 0 to 75, and also the radicals
R1 independently of one another are identical or different and from the group of saturated or unsaturated, optionally branched alkyl groups having 1 to 30 carbon atoms, alkaryl radicals having 7 to 30 carbon atoms, aryl radicals having 6 to 30 carbon atoms, preferably alkyl groups having 1 to 4 carbon atoms or phenyl,
R4 independently of one another are identical or different ester radicals of the general formula IIIa 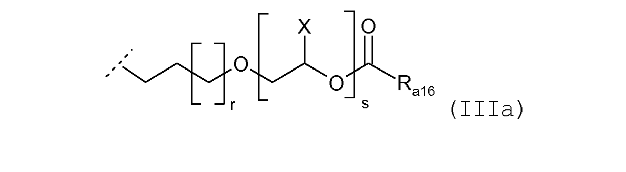
with
r being 1 and
s being 1 or
r being 3 and
s being 0 and
X independently at each occurrence being identical or different radicals from the group -H, -CH₃, -CH₂CH₃ or phenyl, preferably hydrogen,
Rₐ₁₆ being the alkyl radicals of mono-basic fatty acids based on natural vegetable or animal oils having 6 to 30 carbon atoms, in particular having 8 to 22 carbon atoms,
R5 independently at each occurrence being saturated or unsaturated, optionally branched alkyl groups having 1 to 30 carbon atoms or alkaryl radicals having 7 to 30 carbon atoms, preferably alkyl groups having 6 to 22 carbon atoms, or R5 are the radicals of the general formula IIIb 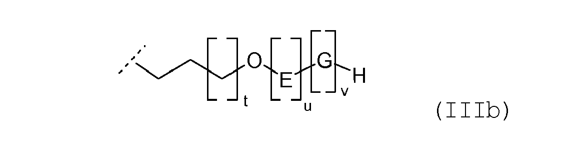 with
t being 1 to 28,
u being 1 to 100,
E being an oxyalkenyl radical of the general formula Ia,
v being 0 to 20,
G if v = 1 being a glycerol-derived oxyalkenyl radical of the general formula Ib, or G if v = 1 being the radical of the general formula Ic with Y and Z independently of one another being identical or different radicals from the group -H, -CH₃, -CH₂CH₃ or -CH₂OH, or G if v ≥ 2 being a polyglycerol-derived radical,
R6 independently at each occurrence being R1, R4 or R5, where R1, R4 and R5 are defined as above and R6 being the same as R4 if e = 0,
R7 independently at each occurrence being identical or different radicals of the general formula IIIc 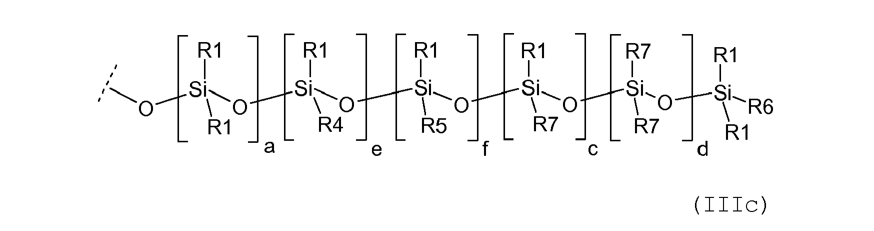
where
R1, R4, R5 and R6 are defined as above.

14. Compound according to Claim 13, **characterized in**
**that**
t is 1 to 28, in particular 1, and
u is 1 to 100, in particular 1 to 20, with particular preference 1, and
v is 0.

15. Compound according to Claim 13, **characterized in**
**that**
t is 1 to 28, in particular 1, and
u is 0, and
v is 1 to 20, in particular 1 to 6, with particular preference 1.

16. Compound of the general formula III, 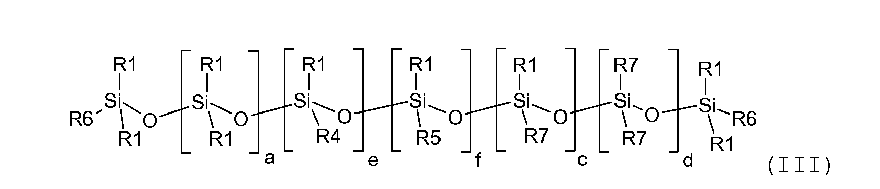 **characterized in that**
N a + b + c + d + e + f + 2 - 3 to 850, preferably 6 to 160,
a is 1 to 800, preferably 2 to 150,
c is 0 to 10, preferably 0,
d is 0 to 10, preferably 0, and
e is 0 to 400, preferably 2 to 75, and
f is 0, and also the radicals
R1 independently of one another are identical or different and from the group of saturated or unsaturated, optionally branched alkyl groups having 1 to 30 carbon atoms, alkaryl radicals having 7 to 30 carbon atoms, aryl radicals having 6 to 30 carbon atoms, preferably alkyl groups having 1 to 4 carbon atoms or phenyl,
R4 independently of one another are identical or different ester radicals of the general formula IIIa 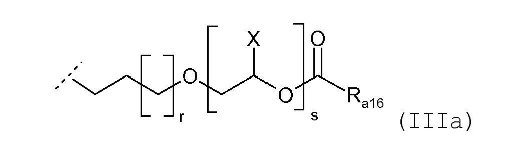 with
r being 1 and
s being 2 to 100, preferably 3 to 80, and
X independently at each occurrence being identical or different radicals from the group -H, -CH₃, -CH₂CH₃ or phenyl, preferably hydrogen
Rₐ₁₆ or -CH₃, with particular preference hydrogen, being the alkyl radicals of mono-basic fatty acids based on natural vegetable or animal oils having 6 to 30 carbon atoms, in particular having 8 to 22 carbon atoms,
R6 independently at each occurrence being R1 or R4, where R1 and R4 are defined as above and R6 being the same as R4 if e = 0,
R7 independently at each occurrence being identical or different radicals of the general formula IIIc 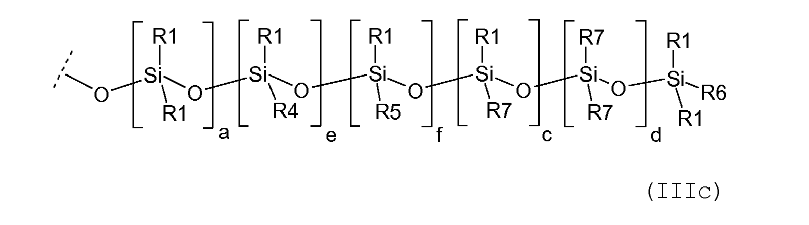
where
R1, R4, R5 and R6 are defined as above.

17. Compound obtained by hydrosilylating esters of the
general formula I 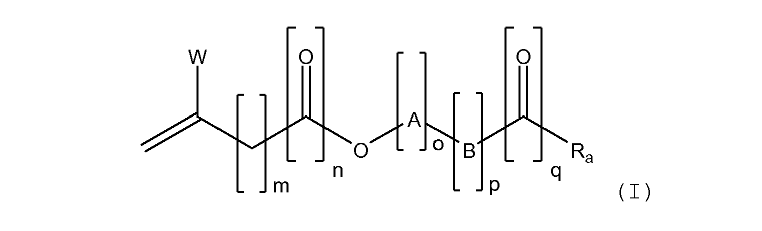 **characterized in that**
W is hydrogen or methyl,
m is 0 to 28, preferably 1 to 17,
n is 0 or 1,
A is oxyalkenyl radical of the general formula Ia 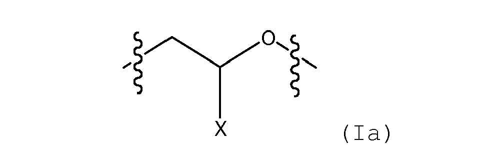 where
X radicals independently are identical or different radicals from the group -H, -CH₃, -CH₂CH₃ or -phenyl, preferably hydrogen,
o is from 0 to 100,
p is 0,
q is 0 or 1,
n + q is 1,
Rₐ = Rₐ₂ or Rₐ₃,
Rₐ₂ is radical of the general formula Id 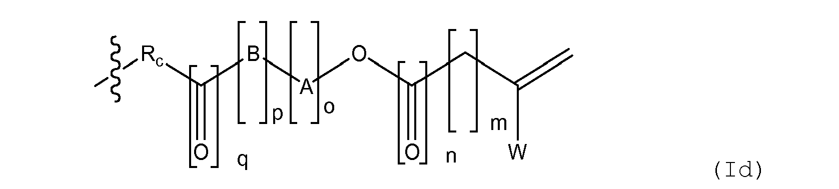
R_{c} is saturated or unsaturated difunctional hydro- carbon radical having 2 to 20 carbon atoms,
Rₐ₃ is linear or branched terminally unsaturated alkyl radical having 2 to 30 carbon atoms, preferably 3 to 30 carbon atoms, which optionally carries hydroxyl groups and/or hydroxyl groups esterified with carboxylic acids, and/or further multiple bonds,
with siloxanes of the general formula V 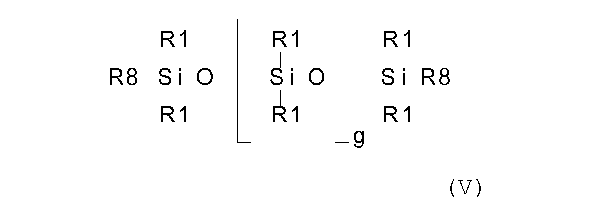
where
g is 3 to 400,
R1 independently at each occurrence is identical or different and from the group of saturated or unsaturated, optionally branched alkyl groups having 1 to 30 carbon atoms, alkaryl radicals having 7 to 30 carbon atoms, aryl radicals having 6 to 30 carbon atoms,
R8 independently at each occurrence is R1 or hydrogen, with the proviso that on average the majority of radicals are hydrogen,
in the presence of a hydrosilylation-active catalyst, with the proviso that the molar ratio of the SiH functions of the siloxane to the double bonds of the ester is 0.8 to 1.2.

18. Compound according to Claim 17, **characterized in that**
W is hydrogen,
m is 0 to 28, preferably 1 to 17,
n is 1,
o is 0,
p is 0,
q is 0,
Rₐ is Rₐ₂.

19. Compound according to Claim 17, **characterized in that**
W is hydrogen,
m is 0 to 28, preferably 1 to 17,
n is 0,
o is 0,
p is 0,
q is 1,
Rₐ is Rₐ₂.

20. Compound according to Claim 17, **characterized in that**
W is hydrogen,
m is 0 to 28, preferably 1 to 17,
n is 0,
o is 1 to 100,
X is hydrogen or methyl, preferably hydrogen,
p is 0,
q is 1,
Rₐ is Rₐ₂.

21. Compound according to Claim 17, **characterized in that**
W is hydrogen,
m is 0 to 28, preferably 1 to 17, with particular preference 1,
n is 0,
o is 1 to 100, preferably 3 to 100,
X is hydrogen or methyl, preferably hydrogen,
p is 0,
q is 1,
Rₐ is Rₐ₃.

## Revendications

1. Procédé pour la préparation de siloxanes modifiés avec des esters organiques, par hydrosilylation de siloxanes avec des esters à insaturation terminale, **caractérisé en ce qu'**on prépare les esters à insaturation terminale utilisés en employant comme catalyseur au moins une enzyme.

2. Procédé selon la revendication 1, **caractérisé en ce qu'**on utilise comme esters à insaturation terminale des composés de formule générale I 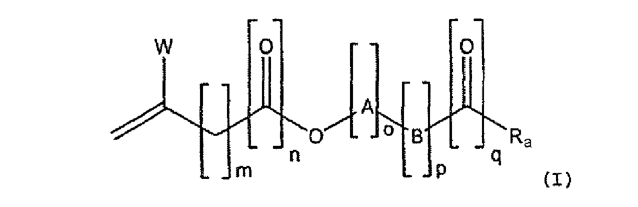 dans laquelle
W représente un atome d'hydrogène ou le groupe méthyle, de préférence un atome d'hydrogène,
m vaut de 0 à 28, de préférence de 1 à 17,
n est 0 ou 1,
o vaut de 0 à 100,
A est un radical oxyalcényle de formule générale Ia 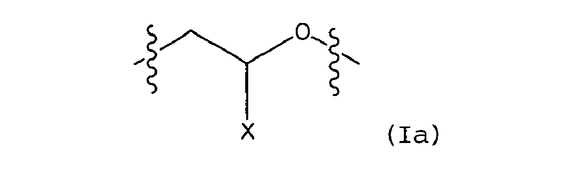 dans laquelle
X représente des radicaux identiques ou différents, choisis indépendamment les uns des autres dans l'ensemble constitué par -H, -CH₃, -CH₂CH₃ ou -phényle, de préférence des atomes d'hydrogène,
p vaut de 0 à 20,
B représente, pour p = 1, un radical oxyalcényle dérivé du glycérol, éventuellement estérifié par R_{b}, de formule générale Ib, 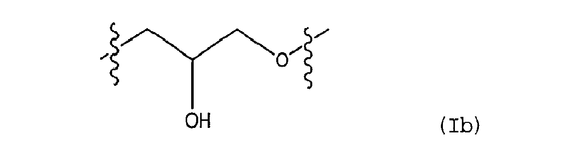
R_{b} étant le radical acyle d'acides carboxyliques linéaires, ramifiés, saturés ou insaturés, ayant de 2 à 30 atomes de carbone, portant éventuellement des groupes hydroxy supplémentaires, ou
B représente, pour p = 1, le radical de formule générale Ic, 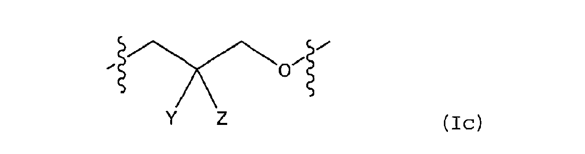
Y et Z représentant, indépendamment l'un de l'autre, des radicaux identiques ou différents, choisis dans l'ensemble constitué par -H, -CH₃, -CH₂CH₃, -CH₂OH ou -CH₂OR_{b}, R_{b} étant tel que défini plus haut, ou
B représente, pour p ≥ 2, un radical dérivé du poly- glycérol, éventuellement estérifié par R_{b}, R_{b} étant tel que défini plus haut,
Rₐ est un atome d'hydrogène, Rₐ₁ ou Rₐ₂,
Rₐ₁ étant un radical alkyle linéaire ou ramifié, saturé ou insaturé, ayant de 1 à 200 atomes de carbone, portant éventuellement des groupes hydroxy, portant éventuellement des groupes hydroxy estérifiés par des acides carboxyliques, portant éventuellement des
Rₐ₂ groupes amino, alylamino ou dialkylamino, étant le radical de formule générale Id 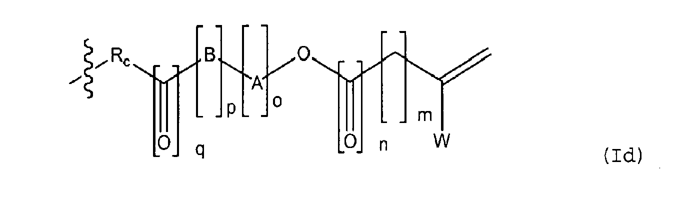 et
R_{c} étant un radical hydrocarboné bifonctionnel saturé ou insaturé ayant de 2 à 20 atomes de carbone,
n + q étant = 1 lorsque
Rₐ est un atome d'hydrogène
q étant 0 et
o + p étant ≥ 1.

3. Procédé selon la revendication 2, **caractérisé en ce qu'**on utilise des esters de formule générale I,
dans lesquels
n, o, p valent 0,
W représente un atome d'hydrogène,
m vaut de 1 à 28, de préférence de 1 à 4,
q est égal à 1,
Rₐ, Rₐ₁ = Rₐ₁₁ ou Rₐ = Rₐ₂,
Rₐ₂ étant tel que défini dans la revendication 2, et
Rₐ₁₁ représentant le radical alkyle dérivé d'acides du commerce, comme par exemple l'acide acétique, l'acide propionique, l'acide butanoïque, l'acide pentanoïque, l'acide chloracétique, l'acide trifluoroacétique, l'acide éthylhexanoïque, l'acide isononanoïque, l'acide isotridécanoïque ou l'acide isostéarique ou le radical alkyle dérivé d'acides gras monobasiques provenant d'huiles naturelles végétales ou animales, ayant de 6 à 30 atomes de carbone, en particulier ayant de 8 à 22 atomes de carbone, tels que l'acide caproïque, l'acide caprylique, l'acide caprique, l'acide laurique, l'acide myristique, l'acide palmitique, l'acide palmitoléique, l'acide isostéarique, l'acide stéarique, l'acide 12-hydroxystéarique, l'acide dihydroxystéarique, l'acide oléique, l'acide linoléique, l'acide pétrosélique, l'acide élaïdique, l'acide arachidique, l'acide béhénique, l'acide érucique, l'acide gadoléique, l'acide linolénique, l'acide eicosapentaénoïque, l'acide docosahexaénoïque, l'acide arachidonique, qui peuvent être utilisés seuls ou en mélange, ou le radical alkyle dérivé de produits de polycondensation d'acides fonctionnalisés par hydroxy, par exemple l'acide poly-12- hydroxystéarique ou l'acide polyricinoléique.

4. Procédé selon la revendication 2, **caractérisé en ce qu'**on utilise des esters de formule générale I, dans lesquels
n, p valent 0
W est un atome d'hydrogène,
m vaut de 1 à 28, de préférence de 1 à 4, en particulier 1,
o vaut de 1 à 100, en particulier 1,
q est égal à 1,
Rₐ, Rₐ₁ = Rₐ₁₁ ou Rₐ = Rₐ₂, Rₐ₁₁ étant tel que défini dans la revendication 3.

5. Procédé selon la revendication 2, **caractérisé en ce qu'**on utilise des esters de formule générale I, dans lesquels
n, o valent 0
m W est un atome d'hydrogène, vaut de 1 à 28, de préférence de 1 à 4, en particulier 1,
B représente -CH₂-CH₂OH-CH₂-O- ou -CH₂-CH₂OR_{b}-CH₂-O-, R_{b} étant tel que défini dans la revendication 2,
p vaut de 1 à 20, de préférence 1,
q est égal à 1,
Rₐ, Rₐ₁ = Rₐ₁₁ ou Rₐ = Rₐ₂, Rₐ₁₁ étant tel que défini dans la revendication 3.

6. Procédé selon la revendication 2, **caractérisé en ce qu'**on utilise des esters de formule générale I, dans lesquels
n, o valent 0
W est un atome d'hydrogène,
m vaut de 1 à 28, de préférence de 1 à 4, en particulier 1,
B représente -CH₂-CH₂YZ-CH₂-O-,
p, q = 1,
Rₐ, Rₐ₁ = Rₐ₁₁ ou Rₐ = Rₐ₂,
Rₐ₁₁ étant tel que défini dans la revendication 3.

7. Procédé selon la revendication 2, **caractérisé en ce qu'**on utilise des esters de formule générale I, dans lesquels
n est égal à 1,
W est un atome d'hydrogène ou CH₃, de préférence un atome d'hydrogène,
m vaut de 0 à 27, de préférence de 1 à 10,
o, p valent 0,
Rₐ, Rₐ₁ = Rₐ₁₂ ou Rₐ = Rₐ₁,
Rₐ₁₂ étant le radical hydrocarboné d'un alcool substitué ou non substitué, éventuellement ramifié, ayant de 2 à 30 atomes de carbone, de préférence ayant de 6 à 22 atomes de carbone, contenant éventuellement une ou plusieurs liaison(s) multiple(s), portant éventuellement des groupes hydroxy, éventuellement des groupes hydroxy estérifiés par des acides carboxyliques, éventuellement des groupes amino, alkylamino ou dialkylamino.

8. Procédé selon l'une quelconque des revendications 1 à 7, **caractérisé en ce que** pour la préparation des esters on utilise au lieu des acides, pour une transestérification, les esters correspondants d'alcools très volatils, de préférence les esters méthyliques, éthyliques et vinyliques.

9. Procédé selon l'une quelconque des revendications 1 à 8, **caractérisé en ce qu'**on utilise des enzymes choisies dans le groupe des enzymes hydrolytiques, par exemple des lipases, estérases ou protéases, de préférence des lipases, de façon particulièrement préférée la lipase B de *Candida antarctica.*

10. Procédé selon l'une quelconque des revendications 1 à 9, **caractérisé en ce qu'**on utilise comme SiH-polysiloxane un composé de formule générale II 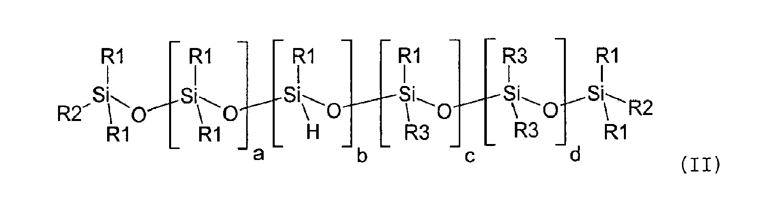 dans laquelle
N a + b + c + d + 2 = 3 à 850, de préférence 6 à 160,
a vaut de 1 à 800, de préférence de 2 à 150,
b vaut de 0 à 400, de préférence de 2 à 75,
c vaut de 0 à 10, de préférence 0,
d vaut de 0 à 10, de préférence 0, et
dans laquelle les radicaux
R1 sont indépendamment les uns des autres identiques ou différents et choisis dans l'ensemble suivant constitué par des groupes alkyle saturés ou insaturés, éventuellement ramifiés, ayant de 1 à 30 atomes de carbone, des radicaux alkaryle ayant de 7 à 30 atomes de carbone, des radicaux aryle ayant de 6 à 30 atomes de carbone, de préférence des groupes alkyle ayant de 1 à 4 atomes de carbone ou phényle, en particulier méthyle,
R2 représentent, indépendamment l'un de l'autre, un atome d'hydrogène ou R1,
R3 représentent chacun indépendamment des radicaux identiques ou différents de formule générale IIa.

11. Composés de formule générale I, **caractérisés en ce que** n = p = 0, W = hydrogène, X = hydrogène ou méthyle, de préférence hydrogène, m = q = 1, o = 2 à 100, de préférence 3 à 100, et Rₐ = Rₐ₁₄, Rₐ₁₄ étant le radical alkyle d'acides du commerce ayant de 4 à 30 atomes de carbone ou d'acides gras monobasiques provenant d'huiles naturelles végétales ou animales, ayant de 6 à 30 atomes de carbone, qui peuvent être présents seuls ou en mélange, ou de produits de polycondensation d'acides fonctionnalisés par hydroxy.

12. Composés de formule générale I, **caractérisés en** c e que n = o = 0, w = hydrogène, m = p = q = 1, B = -CH₂-CH₂OH-CH2-O-, et Rₐ = Rₐ₁₅, Rₐ₁₅ étant le radical alkyle de l'acide myristique ou de l'acide gras de coco.

13. Composés de formule générale III 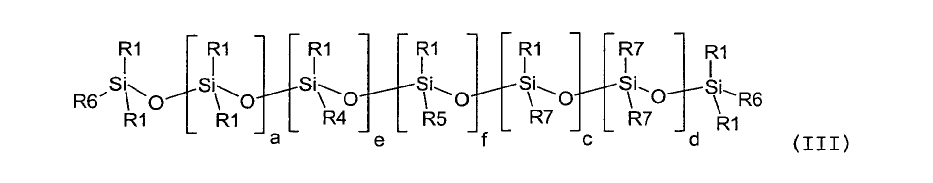 **caractérisés en ce que**
N a + c + d + e + f + 2 = 3 à 850, de préférence 6 à 160,
a vaut de 1 à 800, de préférence de 2 à 150,
c vaut de 0 à 10, de préférence 0,
d vaut de 0 à 10, de préférence 0,
e vaut de 0 à 400, de préférence de 2 à 75 et
f vaut de 0 à 400, de préférence de 0 à 75, ainsi que les radicaux
R1 sont indépendamment les uns des autres identiques ou différents et choisis dans l'ensemble constitué par des groupes alkyle saturés ou insaturés, éventuellement ramifiés, ayant de 1 à 30 atomes de carbone, des radicaux alkaryle ayant de 7 à 30 atomes de carbone, des radicaux aryle ayant de 6 à 30 atomes de carbone, de préférence des groupes alkyle ayant de 1 à 4 atomes de carbone ou phényle,
R4 représentent, indépendamment les uns des autres, des radicaux ester identiques ou différents, de formule générale IIIa 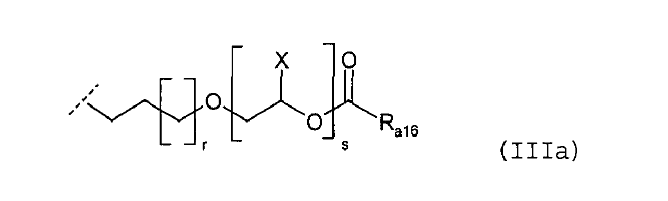 où
r est égal à 1 et
s est égal à 1 ou
r est égal à 3 et
s est égal à 0 et
X représentent, indépendamment les uns des autres, des radicaux identiques ou différents, choisis dans l'ensemble constitué par -H, -CH₃, -CH₂CH₃ ou -phényle, de préférence un atome d'hydrogène,
Rₐ₁₆ représentent les radicaux alkyle d'acides gras monobasiques provenant d'huiles naturelles végétales ou animales, ayant de 6 à 30 atomes de carbone, en particulier ayant de 8 à 22 atomes de carbone,
R5 représentent, indépendamment les uns des autres, des groupes alkyle saturés ou insaturés, éventuellement ramifiés, ayant de 1 à 30 atomes de carbone, ou des radicaux alkaryle ayant de 7 à 30 atomes de carbone, de préférence des groupes alkyle ayant de 6 à 22 atomes de carbone ou R5 représentent les radicaux de formule générale IIIb 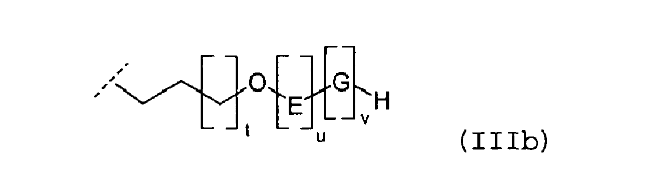 où
t vaut de 1 à 28,
u vaut de 0 à 100,
E est un radical oxyalcényle de formule générale Ia,
v vaut de 0 à 20,
G pour v = 1, représente un radical oxyalcényle dérivé du glycérol, de formule générale Ib, ou G pour v = 1, représente le radical de formule générale Ic où Y et Z représentent, indépendamment l'un de l'autre, des radicaux identiques ou différents, choisis dans l'ensemble constitué par -H, -CH₃, -CH₂CH₃ ou -CH₂OH, ou G pour v ≥ 2, représente un radical dérivé du polyglycérol,
R6 représentent, indépendamment les uns des autres, R1, R4 ou R5, R1, R4 et R5 étant tels que définis plus haut et R6 étant identique à R4 lorsque e = 0,
R7 représentent, indépendamment les uns des autres, des radicaux, identiques ou différents, de formule générale IIIc, 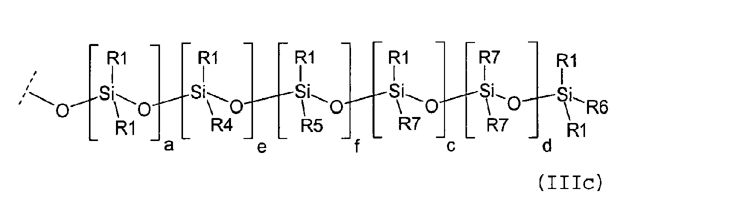
dans laquelle
R1, R4, R5 et R6 sont tels que définis plus haut.

14. Composés selon la revendication 13, **caractérisés en ce que**
t vaut de 1 à 28, en particulier 1 et
u vaut de 1 à 100, en particulier de 1 à 20, de façon particulièrement préférée 1 et
v est égal à 0.

15. Composés selon la revendication 13, **caractérisés en ce que**
t vaut de 1 à 28, en particulier 1 et
u est égal à 0 et
v vaut de 1 à 20, en particulier de 1 à 6, de façon particulièrement préférée 1.

16. Composés de formule générale III 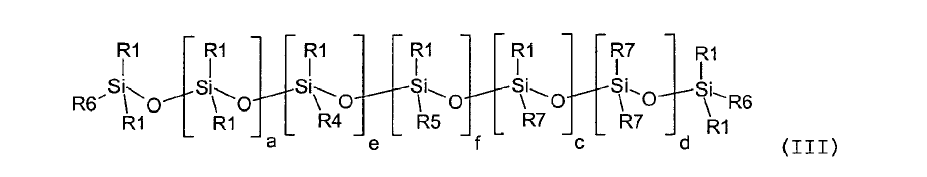 **caractérisés en ce que**
N a + c + d + e + f + 2 = 3 à 850, de préférence 6 à 160,
a vaut de 1 à 800, de préférence de 2 à 150,
c vaut de 0 à 10, de préférence 0,
d vaut de 0 à 10, de préférence 0,
e vaut de 0 à 400, de préférence de 2 à 75,
f est égal à 0, ainsi que les radicaux
R1 sont indépendamment les uns des autres identiques ou différents et choisis dans l'ensemble constitué par des groupes alkyle saturés ou insaturés, éventuellement ramifiés, ayant de 1 à 30 atomes de carbone, des radicaux alkaryle ayant de 7 à 30 atomes de carbone, des radicaux aryle ayant de 6 à 30 atomes de carbone, de préférence des groupes alkyle ayant de 1 à 4 atomes de carbone ou phényle,
R4 représentent, indépendamment les uns des autres, des radicaux ester identiques ou différents, de formule générale IIIa 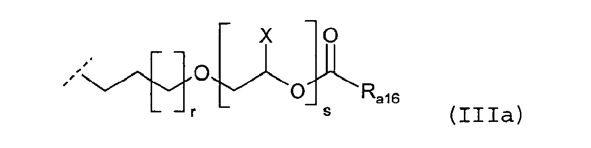 où
r est égal à 1 et
s vaut de 2 à 100, de préférence de 3 à 80 et
X représentent, indépendamment les uns des autres, des radicaux identiques ou différents, choisis dans l'ensemble constitué par -H, -CH₃, -CH₂CH₃ ou -phényle, de préférence des atomes d'hydrogène ou -CH₃, de façon particulièrement préférée des atomes d'hydrogène,
Rₐ₁₆ représentent les radicaux alkyle d'acides gras monobasiques provenant d'huiles naturelles végétales ou animales, ayant de 6 à 30 atomes de carbone, en particulier ayant de 8 à 22 atomes de carbone,
R6 représentent, indépendamment les uns des autres, R1 ou R4, R1 et R4 étant tels que définis plus haut et R6 étant identique à R4 lorsque e = 0,
R7 représentent, indépendamment les uns des autres, des radicaux, identiques ou différents, de formule générale IIIc, 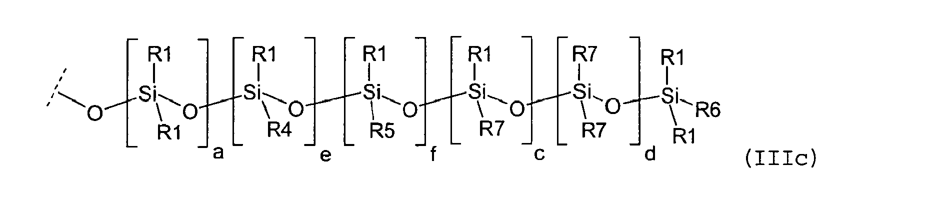
dans laquelle R1, R4, R5 et R6 sont tels que définis plus haut.

17. Composés, qui peuvent être obtenus par l'hydrosilylation d'esters de formule générale I, 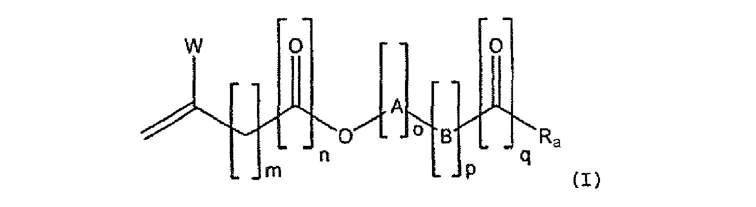 **caractérisés en ce que**
W représente un atome d'hydrogène ou le groupe méthyle,
m vaut de 0 à 28, de préférence de 1 à 17,
n vaut 0 ou 1,
A représente un radical oxyalcényle de formule générale Ia 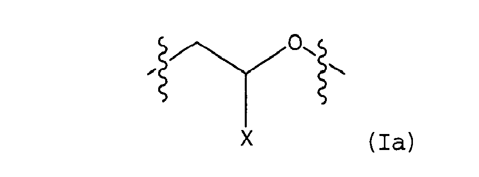 dans laquelle
X représentent, indépendamment les uns des autres, des radicaux identiques ou différents, choisis dans l'ensemble constitué par -H, -CH₃, -CH₂CH₃ ou -phényle, de préférence un atome d'hydrogène,
q
o vaut de 0 à 100,
p est égal à 0
q vaut 0 ou 1,
n + q vaut 1,
Rₐ = Rₐ₂ ou Rₐ₃,
Rₐ₂ représente le radical de formule générale Id 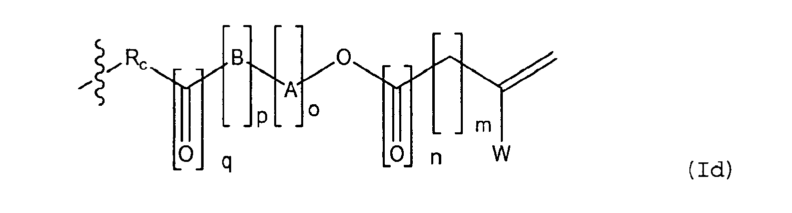
R_{c} représente un radical hydrocarboné bifonctionnel saturé ou insaturé, ayant de 2 à 20 atomes de carbone,
Rₐ₃ représente un radical alkyle linéaire ou ramifié, à insaturation terminale, ayant de 2 à 30 atomes de carbone, de préférence de 3 à 30 atomes de carbone, qui porte éventuellement des groupes hydroxy et/ou des groupes hydroxy estérifiés par des acides carboxyliques et/ou d'autres liaisons multiples,
avec des siloxanes de formule générale V, 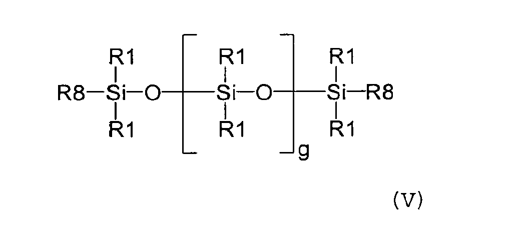 dans laquelle
g vaut de 3 à 400,
R1 sont identiques ou différents et choisis, indépendamment les uns des autres, dans l'ensemble constitué par des groupes alkyle saturés ou insaturés, éventuellement ramifiés, ayant de 1 à 30 atomes de carbone, des radicaux alkaryle ayant de 7 à 30 atomes de carbone, des radicaux aryle ayant de 6 à 30 atomes de carbone,
R8 représentent, indépendamment l'un de l'autre, R1 ou un atome d'hydrogène, étant entendu qu'en moyenne plusieurs radicaux sont des atomes d'hydrogène,
en présence d'un catalyseur réactif pour l'hydrosilylation, le rapport molaire des fonctions SiH du siloxane aux doubles liaisons de l'ester valant de 0,8 à 1,2.

18. Composés selon la revendication 17, **caractérisés en ce que**
m W est un atome d'hydrogène, vaut de 0 à 28, de préférence de 1 à 17,
n est égal à 1,
o est égal à 0,
p est égal à 0,
q est égal à 0,
Rₐ est identique à Rₐ₂.

19. Composés selon la revendication 17, **caractérisés en ce que**
m W est un atome d'hydrogène, vaut de 0 à 28, de préférence de 1 à 17,
n est égal à 0,
o est égal à 0,
p est égal à 0,
q est égal à 1,
Rₐ est identique à Rₐ₂.

20. Composés selon la revendication 17, **caractérisés en ce que**
W est un atome d'hydrogène,
m vaut de 0 à 28, de préférence de 1 à 17,
n est égal à 0,
o vaut de 1 à 100,
X représente un atome d'hydrogène ou le groupe méthyle, de préférence un atome d'hydrogène,
p est égal à 0,
q est égal à 1,
Rₐ est identique à Rₐ₂.

21. Composés selon la revendication 17, **caractérisés en ce que**
W est un atome d'hydrogène,
m vaut de 0 à 28, de préférence de 1 à 17, de façon particulièrement préférée 1,
n est égal à 0,
o vaut de 1 à 100, de préférence de 3 à 100,
X représente un atome d'hydrogène ou le groupe méthyle, de préférence un atome d'hydrogène,
p est égal à 0,
q est égal à 1,
Rₐ est identique à Rₐ₁.
